# FASCICULE DE BREVET EUROPEEN

(11) **EP 1 527 161 B1**
(45) Date de publication et mention de la délivrance du brevet: **28.10.2015**
(21) Numéro de dépôt: 03766432.3
(22) Date de dépôt: 31.07.2003
(51) Int. Cl.: C12N 5/0775, A61K 35/12, G01N 33/50

(54) **CELLULES SOUCHES ISSUES DE TISSU ADIPEUX ET CELLULES DIFFERENCIEES ISSUES DE CES CELLULES**
STAMMZELLEN AUS FETTGEWEBE UND DIFFERENZIERTE ZELLEN DIE DAVON ABSTAMMEN
STEM CELLS DERIVED FROM ADIPOUS TISSUE AND DIFFERENTIATED CELLS DERIVED FROM SAID CELLS

(30) Priorité: 31.07.2002 FR 0209799; 28.02.2003 FR 0302657
(43) Date de publication de la demande: 04.05.2005
(73) Titulaire: Yves Saint-Laurent Parfums, 92951 Neuilly-sur-Seine Cedex (FR); Centre National de la Recherche Scientifique (CNRS), 75794 Paris Cedex 16 (FR); Universite De Nice-Sophia Antipolis, 06108 Nice Cedex 02 (FR)
(72) Inventeur: DANI, Christian, F-06100 Nice (FR); AILHAUD, Gérard, Quartier de la Rouvière, 83590 Gonfaron (FR); RODRIGUEZ, Anne-Marie, 06100 Nice (FR)
(74) Mandataire: Almond-Martin, Carol
(86) Numéro de dépôt international: PCT/FR2003/002439
(87) Numéro de publication internationale: WO 2004/013275

(56) Documents cités:
- WO-A-00/53795
- WO-A-01/11011
- ZUK P A ET AL: "Multilineage cells from human adipose tissue: Implications for cell-based therapies" TISSUE ENGINEERING, LARCHMONT, NY, US, vol. 7, no. 2, avril 2001 (2001-04), pages 211-228, XP002198710 ISSN: 1076-3279
- DATABASE BIOSIS [en ligne] BIOSCIENCES INFORMATION SERVICE, PHILADELPHIA, PA, US; Database accession no. PREV200200368421 XP002249631 & LEE RH ET AL., : "Multipotent mesenchymal stem cells from adult human adipose tissue and femur bone marrow" FASEB JOURNAL, vol. 16, no. 4, - 20 mars 2002 (2002-03-20) page A425
- ZUK PATRICIA A ET AL: "Human adipose tissue is a source of multipotent stem cells." MOLECULAR BIOLOGY OF THE CELL, vol. 13, no. 12, 20 décembre 2002 (2002-12-20), pages 4279-4295, XP002249630 ISSN: 1059-1524
- REYES M ET AL: "PURIFICATION AND EX VIVO EXPANSION OF POSTNATAL HUMAN MARROW MESODERMAL PROGENITOR CELLS", BLOOD, AMERICAN SOCIETY OF HEMATOLOGY, US LNKD- DOI:10.1182/BLOOD.V98.9.2615, vol. 98, no. 9, 1 November 2001 (2001-11-01), pages 2615-2629, XP001153222, ISSN: 0006-4971
- JIANG Y ET AL: "PLURIPOTENCY OF MESENCHYMAL STEM CELLS DERUVED FROM ADULT MARROW", NATURE, NATURE PUBLISHING GROUP, LONDON, GB LNKD- DOI:10.1038/NATURE00870, vol. 418, no. 6893, 4 July 2002 (2002-07-04), pages 41-49, XP001204372, ISSN: 0028-0836
- RODRIGUEZ A-M ET AL: "Transplantation of a multipotent cell population from human adipose tissue induces dystrophin expression in the immunocompetent mdx mouse", THE JOURNAL OF EXPERIMENTAL MEDICINE, ROCKEFELLER UNIVERSITY PRESS, US LNKD- DOI:10.1084/JEM.20042224, vol. 201, no. 9, 2 May 2005 (2005-05-02), pages 1397-1405, XP003005684, ISSN: 0022-1007
- KANG M K ET AL: "Replicative senescence of normal human oral keratinocytes is associated with the loss of telomerase activity without shortening of telomeres.", CELL GROWTH & DIFFERENTIATION : THE MOLECULAR BIOLOGY JOURNAL OF THE AMERICAN ASSOCIATION FOR CANCER RESEARCH JAN 1998, vol. 9, no. 1, January 1998 (1998-01), pages 85-95, ISSN: 1044-9523
- PITTENGER M F ET AL: "Multilineage potential of adult human mesenchymal stem cells.", SCIENCE (NEW YORK, N.Y.) 2 APR 1999, vol. 284, no. 5411, 2 April 1999 (1999-04-02), pages 143-147, ISSN: 0036-8075
- PROCKOP D J: "Marrow stromal cells as stem cells for nonhematopoietic tissues.", SCIENCE (NEW YORK, N.Y.) 4 APR 1997, vol. 276, no. 5309, 4 April 1997 (1997-04-04), pages 71-74, ISSN: 0036-8075
- ZIMMERMANN S ET AL: "Lack of telomerase activity in human mesenchymal stem cells.", LEUKEMIA JUN 2003, vol. 17, no. 6, June 2003 (2003-06), pages 1146-1149, ISSN: 0887-6924
- SIMONSEN J L ET AL: "Telomerase expression extends the proliferactive life-span and maintains the osteogenic potential of human bone marrow stromal cells", NATURE BIOTECHNOLOGY, NATURE PUBLISHING GROUP, NEW YORK, NY, US, vol. 20, 1 June 2002 (2002-06-01), pages 592-596, XP002902749, ISSN: 1087-0156, DOI: 10.1038/NBT0602-592
- KATZ ADAM J ET AL: "Cell surface and transcriptional characterization of human adipose-derived adherent stromal (hADAS) cells", STEM CELLS, ALPHAMED PRESS, DAYTON, OH, US , vol. 23, no. 3 1 March 2005 (2005-03-01), pages 412-423, XP002508651, ISSN: 1066-5099, DOI: 10.1634/STEMCELLS.2004-0021 Retrieved from the Internet: URL:10.1634/STEMCELLS.2004-0021

## Description

La présente invention concerne des cellules souches multipotentes humaines, susceptibles d'être isolées à partir de tissus adipeux humains, ainsi que l'utilisation de ces cellules en thérapie et en cosmétologie. L'invention concerne également une méthode permettant d'isoler ces cellules souches à partir de tissu adipeux adulte humain, ainsi qu'une méthode pour les différencier en cellules d'origine endodermique ou ectodermique ou mésodermique. Enfin l'invention concerne des méthodes de criblage permettant d'identifier des agents susceptibles d'exercer un effet, soit sur la différenciation des cellules, soit sur le fonctionnement des cellules différenciées.

La présence de cellules "souches" multipotentes adultes a été mise en évidence dans un grand nombre de tissus, par exemple la moelle osseuse, le sang, le foie, le muscle, le système nerveux, le tissu adipeux. Les cellules "souches" adultes, en théorie capables d'autorenouvellement à l'infini, possèdent une grande plasticité cellulaire, c'est à dire l'aptitude à se différencier en d'autres tissus que ceux auxquels on les croyait destinées. Les propriétés de ces cellules, similaires à celles des cellules "souches" embryonnaires (ES), ouvrent des perspectives thérapeutiques considérables, d'autant plus que leur utilisation ne pose pas les problèmes de compatibilité et d'éthique, rencontrés avec les cellules ES.

Malheureusement, à l'heure actuelle, leur application médicale (transplantation) reste fortement limitée, essentiellement pour deux raisons :
- premièrement, il est très difficile d'isoler ces cellules. En effet, les cellules "souches" sont très rares dans l'organisme et à ce jour on dispose de peu de connaissances sur celles-ci, notamment en termes moléculaires, ce qui rend leur purification impossible directement. Il existe par exemple une méthode d'enrichissement en cellules multipotentes (population appelée «SP » pour « Side Population ») basée sur la capacité à exclure un colorant vital (Goodell MA et a/.(1996), J Exp Med, vol 83, 1797-1806; Zhou S et al. (2001) Nature Medecine, vol 7, 1028-1034). D'autres méthodes impliquent une séléction positive ou négative, basée sur la présence ou l'absence de marqueurs cellulaires. Par exemple, la demande de brevet internationale WO 01/11011 décrit la déplétion de cellules de moelle osseuse en cellules CD45+ glycophorin A+, suivie par la mise en culture es cellules CD45- / GlyA- en présence de facteurs de croissance. Une méthode similaire est décrite par Reyes et al (Blood, Novembre 2001, vol. 98, n° 9, 2615-2625).
- deuxièmement, l'amplification préalable de ces cellules à l'état indifférencié *in vitro* présente un problème majeur.

Si de nombreux investigateurs ont pu mettre en évidence avec succès la présence de cellules humaines multipotentes dans un grand nombre de tissus, aucun d'entre eux n'a pu maintenir ces cellules, *in vitro,* à l'état indifférencié, au-delà de 50 à 80 doublements de population. Or, la capacité d'autorenouvellement est un indice doublement important : d'une part, en raison du nombre très limité de cellules souches présentes naturellement dans les tissus adultes, une quantité de cellules souches suffisante pour une utilisation thérapeutique ne peut être obtenue que si l'on peut les multiplier *in vitro* tout en conservant leur caractéristiques d'origine. D'autre part, la capacité d'autorenouvellement est en relation étroite avec la définition même de la cellule souche véritable (qui est immortelle) et donc peut être indirectement corrélée à une plasticité cellulaire étendue. Il est donc très souhaitable d'isoler des cellules multipotentes ayant une capacité d'autorenouvellement conservée au delà de 100 doublements de population.

La demande de brevet internationale WO 01/11011 (au nom de Furcht, Verfaillie et Reyes) décrit des cellules humaines multipotentes isolées à partir de la moelle osseuse. Ces cellules ont un caryotype normal, un phénotype HLA Classe I négatif, et peuvent être maintenues en culture *in vitro* jusqu'à 40 doublements de population. Néanmoins, quelques rares cellules peuvent atteindre 70 doublements de population. Les auteurs indiquent que ces cellules peuvent se différencier en cellules du lignage mésodermique, par exemple en ostéoblastes, en chondroblastes, en adipocytes et en myocytes, et également en cellules du lignage ectodermique et du lignage endodermique. Ces cellules étant cependant capables d'un nombre de divisions limité, les auteurs suggèrent, pour permettre la production de quantité importante de cellules, d'y introduire un gène hétérologue codant pour la télomérase. Ce gène hétérologue doit être excisé avant toute utilisation en transplantation. Cette technique d'immortalisation réversible demeure néanmoins fortement contestée. En effet, il subsiste un risque de transformation maligne des cellules au cas où l'on introduit une activité télomérase exogène à un niveau d'expression non-physiologique et non-contrôlé par la cellule (Wong Jing et al., Nature, 405, juin 2000, 755-756). Des travaux similaires sont également décrits par Reyes et al (Blood, Novembre 2001, vol. 98, n° 9, 2615-2625).

Jiang et al (Nature, Advance Online Publication 20 June 2002, doi:10.1098/nature00870) décrivent la production de cellules multipotentes de souris et de rat qui peuvent être maintenues en culture in vitro, au-delà de 100 doublements de population. Les auteurs font également référence à une population multipotente humaine qui aurait pu être maintenue in vitro au-delà de 80 doublements de population. Ceci étant, aucune information complémentaire n'est donnée au sujet de ces cellules humaines.

Qu-Petersen et al (J. Cell Biol. 157, 5, 2002, 851-864) rapportent l'obtention de cellules multipotentes murines à partir de tissu musculaire. Ces cellules, appelées « MDSC » (« Muscle Derived Stem Cells »), ont un caryotype normal et présentent une capacité d'autorenouvellement tout en conservant leur multipotentialité pendant environ 30 doublements de population. Elles peuvent se différencier en cellules appartenant à différents lignages. Ceci étant, le caractère multipotent disparaît au-delà de 40 doublements de population, stade auquel les cellules deviennent sénescentes et meurent. Ces travaux suggèrent fortement que ces cellules multipotentes ont un comportement immunologique privilégié. En effet, l'injection de ces cellules dans des muscles de souris dystrophiques, immunologiquement différentes de celles dont sont issues les cellules MDSC, conduit à une régénération importante du muscle, et ce en l'absence de substances immunosuppressives type cyclosporine. De façon surprenante, les auteurs constatent que la transplantation ne provoque pas d'infiltration du tissu par des lymphocytes CD4⁺ et CD8⁺ de la souris greffée. Cette tolérance est expliquée, du moins en partie, par le phénotype MHC Classe I négative des cellules MDSC. Ce travail montre donc que les cellules MDSC ne sont pas reconnues par les lymphocytes T du receveur, qui est pourtant incompatible immunologiquement, et suggèrent ainsi une utilisation adaptée de ces cellules en allo-transplantation. Cette étude n'a pas été étendue à des cellules humaines.

Deux hypothèses peuvent être proposées pour expliquer la capacité limitée d'autorenouvellement présentée par les différentes cellules multipotentes isolées jusqu'à ce jour :
- d'une part on peut supposer que ces diverses études n'ont pas été menées sur des cellules "souches " véritables mais plutôt sur des précurseurs intermédiaires. Cette hypothèse est d'autant plus fondée que les cellules "souches" peuvent être facilement confondues avec les précurseurs en terme de plasticité. De plus, la contamination de la culture par les précurseurs est facilitée par l'abondance de ceux-ci, comparée aux cellules "souches", mais leur durée de vie est par contre limitée ;
- d'autre part, on peut également envisager que les cellules "souches" n'ont pu être maintenues *in vitro* à l'état indifférencié car les conditions de cultures étaient inadaptées.

Il est également à noter que, à ce jour, un grand nombre des méthodes mises en oeuvre pour obtenir des cellules multipotentes, emploie comme source cellulaire, la moelle osseuse. Or, le prélèvement de cellules de moelle osseuse est une opération délicate, impliquant des risques pour le patient, et représentant une source de cellules souches peu abondante. Il s'agit donc d'une technique peu appropriée pour une production de cellules souches à grande échelle.

Plusieurs équipes d'investigateurs ont donc tenté de mettre au point des méthodes permettant d'isoler des cellules multipotentes à partir d'autres tissus plus abondants, et étant dépourvus de risques majeurs pour les patients. De ce point de vue, le tissu adipeux constitue a *priori* une source prometteuse. Ceci étant, jusqu'à ce jour, bien que la présence de cellules multipotentes ait été mise en évidence dans le tissu adipeux humain, les résultats sont relativement décevants. En effet, les populations cellulaires ainsi obtenues sont souvent hétérogènes et ne peuvent être maintenues en culture *in vitro* au-delà de deux ou trois doublements de population. En outre, à ce jour, aucun investigateur n'a rapporté la production, à partir de tissu adipeux humain, de cellules multipotentes ayant un phénotype HLA Classe I négatif. Cette caractéristique, non-requise pour une utilisation en autogreffe, devient absolument indispensable si ces cellules sont destinées à une utilisation thérapeutique plus étendue, en particulier en allo-transplantation.

Par exemple, les demandes de brevet WO 01/62901 (au nom de Artecel Sciences Inc) et EP 1 077 254 (au nom de Zen Bio Inc) décrivent l'obtention, à partir de tissu adipeux, de populations de cellules stromales, à caractère multipotent. Ces populations sont hétérogènes et contiennent entre autres des pericytes, des cellules endothéliales et des cellules de muscle lisse (voir Erickson et al., Biochem. and Biophys. Res. Com. 290, 763-769, (2002)). Leur capacité d'autorenouvellement est extrêmement limitée, et une analyse de l'expression des marqueurs de surface confirme qu'elles sont HLA Classe I positif. Les caractéristiques de ces populations cellulaires sont donc difficilement compatibles avec une utilisation en thérapie.

La demande de brevet américain US 2002/0076400 (Katz et al.) et la demande de brevet internationale WO 00/53795 (au nom de l'University of Pittsburgh et des Regents of the University of Califonia) décrivent également l'obtention de populations cellulaires multipotentes à partir de tissu adipeux humain. Ces populations de cellules peuvent être différenciées en adipocytes, en ostéoblastes, en chondrocytes et en myocytes. Selon les auteurs, elles peuvent être maintenues en culture *in vitro* pendant au moins 15 transferts cellulaires sans perdre leur caractère multipotent. Aucune donnée sur le doublement de population correspondant n'est donnée. Avant de soumettre les cellules à des transferts successifs, une activité télomérase est détectée dans cette population, pourtant hétérogène. Cette activité n'a pas été mesurée après transferts successifs. Aucune analyse de marqueurs de surface n'a été effectuée. L'expression d'antigènes HLA Classe I n'a donc pas été déterminée.

Zuk et al., Tissue Engineering, 7(2) 211-228, (2001) décrit un procédé d'obtention de cellules multipotentes à partir d'un prélèvement de tissu adipeux obtenu par liposuccion (processed lipoaspirate, PLA). Ces cellules présentent cependant un taux de sénescence de 15% au quinzième passage.

La présente invention permet de remédier aux inconvénients des techniques précédemment décrites.

En effet, les présents inventeurs ont mis au point une méthode permettant d'isoler de façon reproductible, des cellules "souches" multipotentes à partir de tissu adipeux de jeunes enfants et de les multiplier, à l'état indifférencié, en grande quantité *in vitro* pendant plus de 200 doublements de population. Leur utilisation thérapeutique devient alors possible. L'invention a pour objet principal, le procédé de production de cellules souches à partir de tissu adipeux, ainsi que l'utilisation des cellules souches obtenues.

Dans le contexte de la présente invention, les termes suivants signifient :
- autorenouvellement :: la capacité de se diviser sans altérer les caractéristiques initiales de la cellule.
- cellule souche :: cellule multipotente ayant une capacité d'autorenouvellement élevée, ayant une activité télomérase, et étant capable de rentrer en quiescence.
- cellule souche adulte :: cellule souche autre qu'une cellule souche embryonnaire, provenant par exemple d'un nouveau né, d'un enfant ou d'un adulte.
- multipotent ou multipotentiel :: capable de se différencier en au moins deux types cellulaires.
- quiescent :: la capacité d'une cellule de rester dans un état de non-prolifération et de non-sénescence.

Plus particulièrement, l'invention concerne un procédé d'obtention de cellules souches humaines multipotentes à partir de tissu adipeux adulte. Ce procédé comprend, comme première étape, la mise en culture de cellules provenant d'un prélèvement de tissu adipeux humain. D'autres types de tissu qui peuvent être utilisés, sans être inclus dans le procédé selon l'invention, incluent les muscles, la moelle osseuse, le foie, le système nerveux. Après 12 heures de culture, les cellules sont séparées en deux sous-populations en fonction de leur vitesse d'adhésion, une première population cellulaire dite « CA » adhérant en moins de 12h, et une deuxième population cellulaire dite « CS » adhérant plus lentement et se trouvant après 12 heures de culture, en suspension dans le milieu de culture. La population « CA » est ensuite enrichie jusqu'à l'obtention d'une population de cellules capables de rentrer dans un état de quiescence. A partir de ce stade, une prolifération intensive des cellules souches de la population « CA » peut être alors induite.

Selon une variante préférée de l'invention, le procédé d'obtention de cellules souches humaines multipotentes, comprend les étapes suivantes :
a) digestion enzymatique d'un prélèvement de tissu adipeux,
b) récupération d'une fraction cellulaire dépourvue d'adipocytes, contenant tous les types cellulaires présents dans la préparation obtenue selon (a), à l'exception des adipocytes,
c) culture *in vitro* pendant au moins 12 heures, de la fraction cellulaire obtenue selon l'étape (b),
d) sélection de deux sous-populations cellulaires, dites population « CA » et population « CS », la population CA présentant une vitesse d'adhésion inférieure à 12 heures, et la population « CS » présentant une vitesse d'adhésion supérieure à 12 heures,
e) enrichissement de la population « CA » jusqu'à l'obtention d'une population de cellules capables de rentrer dans un état de quiescence,
f) éventuellement, induction d'une prolifération accrue des cellules souches de la population « CA », par exemple par addition d'un facteur de croissance.

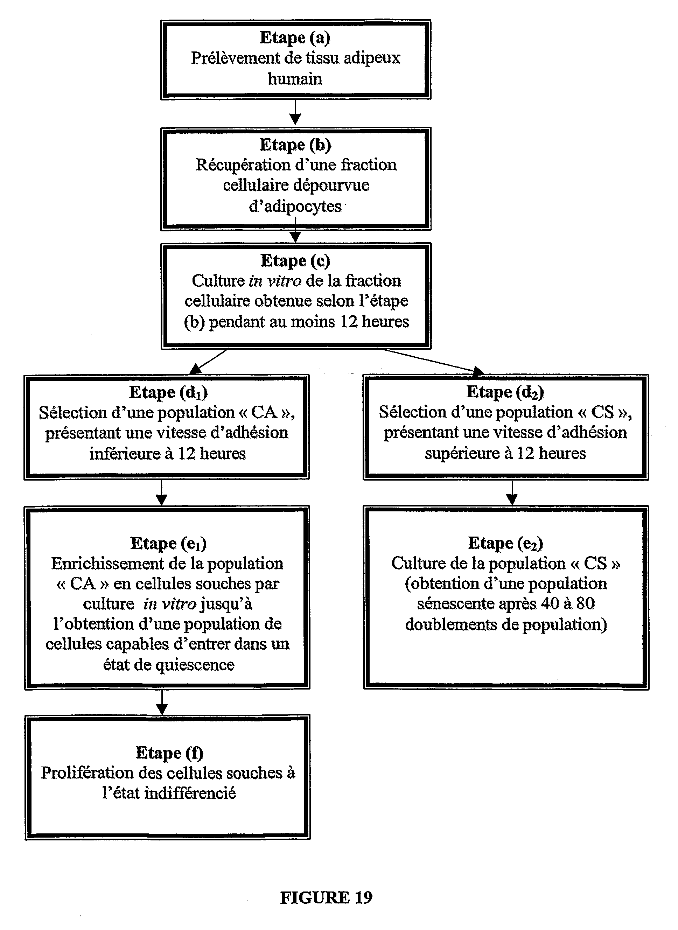

La Figure 19 présente un schéma d'une variante préférée du procédé de l'invention.

### Etape (a) : digestion enzymatique d'un prélèvement de tissu adipeux :

L'étape de digestion enzymatique est de préférence effectuée par mise en contact du prélèvement de tissu adipeux avec une préparation enzymatique telle que la collagénase pendant une durée courte, c'est-à-dire maximum 10 minutes, et plus préférablement de 5 à 10 minutes, ou encore de 5 à 8 minutes. Ceci permet une dissociation complète du tissu tout en évitant d'endommager certains types cellulaires, et conduit donc à une meilleure viabilité de tous les types cellulaires.

En ce qui concerne la nature du tissu adipeux, il provient de préférence d'un individu sain, préférentiellement d'un jeune enfant sain, âgé de préférence de moins de 10 ans, par exemple d'un nouveau-né ou d'un enfant âgé de 2 ou 3 mois à 8 ans. Il peut s'agir d'enfants de sexe masculin ou féminin.

L'âge du donneur semble être un point important. En effet, un certain nombre de données obtenues à partir de cellules "souches" hématopoïétiques suggère fortement que les cellules "souches", non seulement diminuent en nombre avec l'âge de l'individu mais subissent également un processus de vieillissement se traduisant par une perte de fonctionnalité (Geiger H et Van Zant (2002), Nature, vol3, n°4, 329-333).

Si l'on extrapole ces données, le tissu adipeux de jeunes enfants, semble constituer une source de cellules "souches" plus abondantes et plus fonctionnelles que le tissu adipeux d'individus adultes.

Le prélèvement de tissu adipeux peut provenir de n'importe quel site anatomique, mais est préférentiellement un prélèvement de tissu d'origine extramédullaire, provenant plus particulièrement de la région ombilicale ou de la région pubienne ou de la région inguinale ou de la région périnéale ou de la région abdominale ou de la région sous-cutanée. Les régions pubienne, pré-pubienne, inguinale et ombilicale sont plus particulièrement préférées.

### Etape (b) : récupération d'une fraction cellulaire dépourvue d'adipocytes :

Le tissu adipeux ayant subi une digestion enzymatique est ensuite traité pour retirer les adipocytes. On récupère alors une fraction cellulaire dépourvue d'adipocytes, contenant tous les types cellulaires présents dans le tissu adipeux (par exemple préadipocytes, cellules souches, cellules endothéliales, péricytes, mastocytes...), à l'exception des adipocytes.

L'élimination des adipocytes peut être effectuée par tout moyen approprié. La centrifugation est particulièrement efficace, puisque toutes les cellules d'intérêt se trouvent dans le culot de centrifugation tandis que les adipocytes flottent dans le surnageant.

Il est important de noter que cette étape de la procédure est effectuée sans filtration, permettant ainsi de conserver en culture tous les types cellulaires, autres que les adipocytes. En effet, dans les techniques classiquement décrites de préparation cellulaire à partir de tissus adipeux, on procède généralement à des étapes de filtrations successives ou non, selon les auteurs, et ceci avant ou après la centrifugation, afin d'éliminer les déchets. Cette étape cependant risque d'entraîner la perte de certains types cellulaires.

### Etape (c) : culture in vitro :

La fraction cellulaire obtenue lors de l'étape (b) est ensuite mise en culture pendant au moins 12 heures, et de préférence pendant 12 à 80 heures, par exemple 12 à 72 heures.

Pour cette étape de la procédure, l'ensemencement des cellules est effectué à une densité comprise entre 1000 et 5000 cellules /cm², par exemple 1000 à 3500 cellules /cm². En effet, les cellules multipotentes sont peu représentées, en comparaison aux autres types cellulaires, et un ensemencement à haute densité permet donc de s'assurer que chaque boîte contient ce type de cellule.

Le milieu de culture utilisé pour cette étape du procédé est normalement un milieu de culture type DMEM, additionné de sérum foetal sans ajout d'autres facteurs de croissance. Par exemple, un milieu particulièrement approprié est le suivant : DMEM+ 10 % de sérum de veau foetal décomplémenté + antibiotiques (100 U/ml de pénicilline, 100 µg / ml de streptomycine).

Le rendement cellulaire à cette étape est variable selon les prélèvements : de 1000 à 5000 cellules par mg de tissu.

### Etape (d) : sélection de deux sous-populations cellulaires :

L'étape d'enrichissement en cellules souches multipotentes débute par la séparation, au début de la mise en culture selon l'étape (c), de deux sous-populations cellulaires en fonction de leur vitesse d'adhésion :
- une population cellulaire dite CA adhérant en moins de 12h
- une population cellulaire dite CS adhérant plus lentement (de 48 à 72h).

La population CS se trouve après 12 heures de culture, en suspension dans le milieu de culture, alors que la population CA adhère aux boîtes.

Les cellules "souches" se retrouvent uniquement dans la sous-population CA alors que la deuxième sous-population contient des précurseurs multipotents qui meurent après environ 60 doublements de population. Bien que la population CS ne puisse donc être utilisée pour la production de cellules souches, elle peut néanmoins être utilisée pour d'autres applications. En effet la population CS présente les caractéristiques suivantes :
i) elle est multipotente,
ii) elle a un phénotype HLA Classe I négatif,
iii) elle a un caryotype normal,
iv) elle présente une capacité d'autorenouvellement conservée pendant environ 40 à 60 doublements de population
v) sa vitesse de prolifération n'est pas affectée par le « Leukemia Inhibitory Factor » (LIF).

Cette population cellulaire se prête donc à des utilisations thérapeutiques et cosmétiques comparables à celles habituellement connues pour des cellules multipotentes de l'art antérieur.

L'étape de sélection de la population adhérant rapidement (population CA) est importante puisqu'elle permet, dès la mise en culture, de moins diluer les cellules "souches" par rapport à la masse des différentes cellules précurseurs, en faisant une première sélection.

### Etape (e) : enrichissement en cellules souches :

Les populations CA et CS sont ensuite mises en culture dans des conditions identiques. Pour la population CA, cette mise en culture permet d'obtenir un enrichissement important en cellules souches. Cette étape d'enrichissement est basée sur le fait que les précurseurs ont une durée de vie limitée par rapport aux cellules souches (en théorie immortelles). Lors des premiers doublements de population, les précurseurs vont se multiplier beaucoup plus rapidement que les cellules souches, puis ceux-ci vont commencer à mourir, jusqu'au stade 50 à 80 doublements de population. A ce stade, la population est fortement enrichie en cellules souches.

Lors de cette étape, chaque transfert cellulaire est effectué lorsque les cellules arrivent à 80% de confluence et l'ensemencement est effectué à haute densité, c'est-à-dire à une densité comprise entre 1000 à 5000 cellules / cm², de préférence entre 1000 à 3500 cellules / cm², et plus particulièrement entre 2000 et 2500 cellules /cm².

A chaque transfert cellulaire, les cellules sont diluées par 2 ou par 3 au maximum, pendant environ 50-80 doublements de population (stade auquel la population CA est fortement enrichie en cellules "souches" et où la population CS meurt, ce qui correspond à la mort des précurseurs). Cette étape est indispensable si on émet l'hypothèse que la vraie cellule "souche", qui est une cellule quiescente à l'état normal, se divise plus lentement qu'un précurseur. Une dilution supérieure des cellules au cours des étapes de trypsinisation pourrait entrainer le risque de perdre ces cellules multipotentes dans certaines boites de culture.

Après environ 50 à 80 doublements de population (par exemple 60 doublements de population), la population CS présente les caractéristiques d'une population sénescente (perte du potentiel prolifératif et perte de la multipotentialité) et meurt. Par contre la population CA, à ce même stade, prolifère plus lentement comparé aux premiers doublements de population (temps de doublement de population d'environ 72 heures, comparé à un temps moyen de doublement d'environ 36 heures initialement), et est capable de rentrer en quiescence.

La population CA peut être considérée comme ayant atteint la quiescence lorsqu'elle présente les caractéristiques suivantes :
▪ arrêt spontané de la prolifération à 70% de confluence environ ;
▪ la confluence peut être atteinte à ce stade en présence de bFGF ou autres facteurs de croissance. Une diminution du temps de doublement de population de 72 heures environ à 36 heures environ peut être observée ;
▪ l'effet du bFGF ou autres facteurs de croissance sur le temps de doublement est réversible.

En outre, la mesure de l'activité X-gal endogène déterminée à pH 6 dans la population CA est négative (inférieur à 0.05 %), confirmant que cette population est à l'état quiescent et non sénescent.

Le milieu de culture employé pour cette étape d'enrichissement est typiquement un milieu sans facteurs de croissance rajouté, par exemple le milieu de culture DMEM+ 10% de sérum de veau foetal décomplémenté + antibiotiques (100 U/ml de pénicilline, 100 µg/ml de streptomycine).

### Stade (f) : induction de prolifération des cellules souches à l'état indifférencié :

Après avoir atteint la quiescence, la prolifération des cellules de la population CA est induite par trypsinisation et dilution des cellules dans de nouvelles boîtes. De préférence, les cellules sont soumises à un traitement à la trypsine à 80% de confluence et diluées de 2 à 10 fois, de préférence de 5 à 10 fois, dans de nouvelles boites de culture identiques.

L'ajout d'un facteur de croissance à ce stade, par exemple le facteur de croissance fibroblastique basique (bFGF), le PDGF, l'EGF, le NGF ou le SCF permet la prolifération intensive des cellules "souches" de la population CA. L'ajout de bFGF humain à ce stade est particulièrement préféré.

L'addition de facteurs de croissance tels que le bFGF, incorporé par exemple à une concentration d'environ 3 à 20 ng/ml de milieu, particulièrement 5 à 10 ng/ml, permet non seulement de réduire de moitié le temps de doublement de population (par exemple le temps de doublement de population sans bFGF est d'environ 72 heures, alors qu' avec le bFGF il est d'environ 36 heures, mais elle permet également à la population d'atteindre la confluence. En effet sans facteurs de croissance, la prolifération d'une population quiescente de cellules souches de l'invention peut être provoquée par trypsination et dilution, mais la prolifération s'arrêtera spontanément à 70 % de confluence environ. Dans la mesure où la confluence est un état indispensable in vitro pour initier la différenciation de nombreux types cellulaires, l'utilisation de facteurs de croissance tels que le bFGF doit être prévue pour la production *in vitro* de ce type de cellule différenciée.

Cette étape du procédé se distingue clairement des procédés de l'art antérieur. En effet, selon le procédé de l'invention, les facteurs de croissance tels que le bFGF ne sont utilisés qu'après avoir obtenu une population capable de rentrer en quiescence. Par contraste, le bFGF est généralement utilisé dès la mise en culture des cellules fraîchement isolées (Tsutsumi S et al., Biochemical and Biophsysical Research Communications 288,413-419 (2001)). Cette utilisation très précoce du bFGF a un effet pervers car il stimule, non seulement la prolifération des cellules "souches" véritables mais également de tous les précurseurs. Ceci a pour conséquence d'accroître davantage le rapport précurseurs/ cellules "souches", se traduisant par la perte par dilution de ce type cellulaire rare. Selon l'invention, le point novateur est d'utiliser le bFGF lorsque la masse des précurseurs a disparu et que la population, fortement enrichie en cellules "souches", devient quiescente.

L'induction de la prolifération des cellules souches permet de produire des quantités importantes de ces cellules multipotentes. Les cellules ainsi produites peuvent être récupérées des milieux de culture en utilisant des méthodes classiques.

En résumé, le procédé de l'invention comporte plusieurs éléments novateurs permettant d'optimiser la production de cellules souches :
- une digestion rapide par une préparation enzymatique telle que la collagénase (étape (a)) qui permet une dissociation complète du tissu tout en évitant d'endommager certains types cellulaires,
- l'absence d'étapes de filtrations dans l'étape (b), pour éviter de perdre sur les filtres certains types de cellules,
- l'ensemencement des cellules à haute densité lors des étapes de culture (d) et (e). En effet, les cellules multipotentes sont peu représentées, en comparaison aux autres types cellulaires,
- l'isolement de 2 sous-populations « CA » et « CS » en fonction de leur vitesse d'adhésion,
- l'utilisation tardive d'un facteur de croissance tel que le bFGF, après que les cellules soient devenues quiescentes (étape f).

En mettant en oeuvre le procédé de l'invention, les présents inventeurs ont établi plusieurs lignées multipotentes humaines à partir de tissu adipeux de jeunes enfants. La technique de l'invention a été validée sur plusieurs prélèvements de tissus adipeux, par exemple celles indiquées le Tableau I (voir Exemples ci-dessous).

Les cellules de l'invention présentent de nombreuses caractéristiques des cellules souches, par exemple : capacité à rentrer en quiescence ; sortie de la quiescence induite par le bFGF ou autres facteurs de croissance, se traduisant par une reprise intense de la prolifération (effet réversible de ces facteurs de croissance, sécrétés *in vivo* dans l'organisme lors de dommage corporels) ; maintien de la multipotentialité pendant un nombre élevé de doublements de population ; une activité télomérase significative ; un caryotype normal.

Plus particulièrement, l'invention concerne une cellule souche adulte humaine et multipotente isolée, caractérisée en ce qu'elle présente :
i) une capacité d'autorenouvellement conservée pendant au moins 130 doublements de population,
ii) une activité télomérase endogène d'au moins 20% de l'activité télomérase de la lignée humaine transformée HEK293T,
iii) un phénotype H LA Classe I négatif, y compris en présence de 10% de sérum de veau foetal,
iv) un caryotype normal,
v) une capacité à rentrer en quiescence après 50 à 80 doublements de population,
vi) un taux de sénescence inférieur à 0.05% au stade de 60 doublements de population.

La capacité d'autorenouvellement des cellules de l'invention est conservée pendant au moins 130 doublements de population, et plus particulièrement pendant au moins 200 doublements de population. Ceci signifie que les cellules de l'invention sont capables de subir au moins 130 ou 200 doublements de population sans perdre leurs caractéristiques d'origine. En d'autres termes, la multipotentialité, l'activité télomérase, le phénotype HLA Classe I négatif, le caryotype normal et la capacité à entrer en quiescence sont conservés pendant tous ces doublements de population.

L'activité télomérase des cellules de l'invention, mesurable en mettant en oeuvre des techniques conventionnelles, est particulièrement importante. En effet, en conformité avec la définition de la cellule "souche" de Watt et Hogan (Science, vol 287, February 2000), l'activité télomérase signifie normalement que les cellules obtenues seraient capables d'autorenouvellement à l'infini. L'activité télomérase est uniquement présente dans les cellules embryonnaires et, chez l'adulte, dans les cellules tumorales et les cellules "souches". Cette activité confirme donc qu'il s'agit bien de cellules souches.

Le niveau d'activité télomérase endogène des cellules de l'invention correspond à au moins 20 %, par exemple 20 à 50 % de l'activité télomérase d'une lignée cellulaire de référence, plus particulièrement 22 à 50 %. La lignée de référence est la lignée humaine transformée HEK293T (Human Embryonic Kidney 293 immortalisé par l'Ag T), une lignée transformée ayant une activité télomérase endogène. Cette activité peut être mesurée à n'importe quel stade. Elle est de préférence mesurée au-delà de 30 ou 40 doublements de populations, par exemple au stade de la quiescence après environ 60 doublements de populations.

En ce qui concerne les caractéristiques immunologiques des cellules de l'invention, elles sont différentes d'une cellule somatique classique. En effet, ces cellules n'expriment pas de molécules du système HLA de classe I en surface (confirmé par des analyses de cytométrie de flux), ni de HLA Classe II en surface. Les molécules du système HLA de classe I présentent les peptides antigéniques du soi et du non soi aux lymphocytes T cytotoxiques CD8⁺, d'où leur rôle critique dans les réactions de rejet de greffes. L'absence de surface des molécules HLA de classe I laisse supposer « l'universalité » des cellules souches de l'invention en transplantation. Ces cellules pourraient être utilisées en allotransplantation sans aucun risque de rejet de la part de l'hôte, indépendamment de son génotype.

Le phénotype HLA Classe I et / ou Classe II négatif peut être analysé par toute technique conventionnelle. Il est de préférence mesuré au-delà de 30 ou 40 doublements de populations, par exemple au stade de la quiescence vers 60 doublements de populations, ou à un stade tardif c'est-à-dire au-delà de la quiescence, par exemple à 100 ou 120 doublements de population. En effet, pendant les premiers doublements de population, l'expression de surface des molécules HLA de Classe I est faible mais significative, puis disparaît à des stades tardifs (par exemple au-delà de la quiescence entre 50 et 80 doublements de populations), correspondant au stade de la disparition des précurseurs précoces.

Dans le contexte de l'invention, l'expression « HLA négatif » signifie que les cellules souches de l'invention présentent un niveau d'expression de surface de molécules HLA de Classe I non-détectable par cytométrie de flux par simple marquage (utilisation d'un fluorochrome). Préférentiellement, les cellules « HLA négatif » de l'invention présentent également un niveau d'expression de surface de molécules HLA de Classe II non-détectable par cytométrie de flux par simple marquage.

En outre, grâce à un phénomène de comportement immunitaire privilégié, les cellules de l'invention, à l'état différencié, n'induisent vraisemblablement pas de réaction de rejet chez un hôte, indépendamment de son génotype.

Les cellules de l'invention présentent un caryotype normal, confirmant qu'elles ne sont pas transformées.

A l'état normal, les cellules de l'invention sont quiescentes (Blau HM et al, Cell, 105, 829-841, June 29, (2001)) et se remettent à proliférer en présence de bFGF. La quiescence est une caractéristique propre aux cellules souches. En effet, au-delà d'un certain nombre de doublements de populations, les cellules «non-souches » arrivent à la sénescence. L'état de quiescence peut, en théorie, être maintenu indéfiniment. Pour les cellules de l'invention, les inventeurs ont maintenu cet état pendant des périodes allant jusqu'à un an. La prolifération peut ensuite être induite par trypsination et dilution, éventuellement accompagné de l'ajout d'un facteur de croissance. A quiescence, les cellules de l'invention s'arrêtent de proliférer spontanément, avant d'atteindre la confluence, par exemple entre 50% et 90% de confluence, plus particulièrement entre 60 % et 70 % de confluence.

Une caractéristique importante des cellules de l'invention est leur multipotentialité. Elles sont en effet capables de se différencier en au moins deux types cellulaires. Plus particulièrement, elles sont capables de se différencier en cellules d'origine endodermique (par exemple le foie) ou ectodermique (cellules nerveuses : astrocytes, oligodendrocytes et neurones) ou encore mésodermique. Comme exemples de cellules du lignage mésodermique, l'on peut citer les adipocytes, les ostéoblastes, les myocytes, les cellules endothéliales et les chondrocytes.

Il a notamment été démontré que les cellules de l'invention, même à des stades tardifs, sont capables de se différencier en adipocytes fonctionnels (démontré par lipolyse, activité GPDH, marqueurs adipocytaires), en ostéoblastes fonctionnels (démontré par présence de marqueurs ostéoblastiques et calcification de la matrice extracellulaire), en myocytes fonctionnels, et en cellules endothéliales. Cette différenciation peut avoir lieu *in vitro* et *in vivo.*

Il a notamment été démontré que les cellules « CA » de l'invention, ont, au niveau clonal, la capacité de se différencier en adipocytes, en ostéoblastes, en myocytes, et en cellules endothéliales, c'est-à-dire chaque cellule CA de l'invention est capable de se différencier en ces quatre types cellulaires. Contrairement aux cellules adipocytaires, ostéoblastiques et myocytaires qui appartiennent au "Limb bud mesoderm", les cellules endothéliales dérivent du mésoderme viscéral. Les cellules de l'invention ne sont donc pas limitées à une différenciation en cellules du lignage mésenchymateux, mais peuvent se différencier plus généralement en cellules du lignage mésodermique.

Les inventeurs ont investigué la présence de marqueurs sur les cellules souches de l'invention. Ils ont constaté qu'elles expriment les facteurs de transcription Oct-4 et Rex-1, et l'antigène de surface ABCG2 (transporteur ABC responsable du phénotype « SP » : Zhou S et al., Nature Medicine, 7, n° 9, Sept 2001, 1028-1034). Les facteurs de transcription Oct-4 et Rex-1 sont exprimés spécifiquement dans les cellules souches embryonnaires de souris et humaines. Oct-4 est indispensable au maintien de la pluripotentialité des cellules souches embryonnaires de souris. Il a été montré également que Oct-4 est exprimé par les cellules souches embryonnaires humaines.

Il a également été observé que les cellules souches de l'invention ne réagissent pas au « Leukemia Inhibitory Factor » (LIF), à des concentrations d'environ 10ng/ml. Le LIF ne produit aucun changement de morphologie ou de prolifération des cellules. Conformément aux résultats obtenus par d'autres investigateurs avec des cellules souches humaines, notamment des cellules souches embryonnaires, il peut donc être conclu que les cellules de l'invention n'expriment vraisemblablement pas le récepteur pour le LIF (« LIF-R »), et sont donc LIF-R négatif.

De préférence, les cellules de l'invention, après avoir atteint la quiescence, présentent de façon stable le phénotype suivant *in vitro :*
HLA classe I négatif,
HLA classe II négatif,
CD3 négatif,
CD13 positif,
Oct-4 positif,
Rex-1 positif,
ABCG2 positif.

Ces caractéristiques phénotypiques s'associent à un caryotype normal, et à une activité télomérase significative. De préférence les cellules sont également LIF-R négatives. Ce phénotype est conservé in vitro de façon stable, c'est-à-dire en l'absence ou en présence de FGF-2, et à des concentrations de sérum de veau foetal qui peuvent dépasser 10%. Le phénotype est également conservé à des densités d'ensemencement élevées, et au-delà de 140 doublements de populations.

Le temps de doublement des populations cellulaires de l'invention varie en fonction de la proportion de cellules souches présentes. Par exemple, avant d'atteindre la quiescence, le temps de doublement est d'environ 36 à 40 heures, reflétant la présence de précurseurs dans la population CA. Au fur et à mesure que la proportion de cellules souches augmente, le temps de doublement augmente également, pour atteindre environ 70 à 80 heures à quiescence. En effet les cellules souches se divisent beaucoup plus lentement que les précurseurs. Après quiescence, l'ajout de facteurs de croissance tels que le bFGF permet de réduire de façon significative le temps de doublement, par exemple jusqu'à 36 heures environ, permettant une production intensive et rapide de cellules souches.

Les cellules de l'invention peuvent être modifiées génétiquement puis sélectionnées pour introduire ou faire exprimer une caractéristique nouvelle, par exemple par ablation ou modification d'un endogène ou pour l'expression d'un transgène tel qu'un gène rapporteur ou un gène dont le produit d'expression présente des propriétés thérapeutiques, sous le contrôle d'un promoteur approprié, par exemple ubiquiste ou tissu-spécifique.

L'expression d'un transgène ou d'un ADN ou d'un ARN peut être soit constitutive, soit inductible de manière réversible ou irréversible. L'ADN ou l'ARN hétérologue d'intérêt peut être porté par tout vecteur d'expression, par exemple vecteur viral (y compris vecteurs rétroviraux), vecteurs inertes, vecteurs plasmidiques ou encore vecteur épisomal.

L'introduction des vecteurs dans les cellules peut se faire par transfection, par exemple par utilisation d'agents chimiques tels que le phosphate de calcium, par lipofection ou encore par utilisation d'agent physique par exemple l'électroporation, la micro-injection, etc.... Le maintien de ce vecteur dans la cellule peut-être soit sous forme épisomale soit sous forme intégrée dans le génome, au hasard ou de manière ciblée.

De telles cellules génétiquement modifiées peuvent être utilisées en thérapie génique pour permettre d'apporter un produit d'expression d'un gène hétérologue à un individu. Grâce au caractère multipotent et HLA Classe I négatif, les cellules souches de l'invention sont particulièrement appropriées pour ce type d'application.

Lorsque les cellules de l'invention sont transduites ou transfectées par un gène rapporteur, elles peuvent être utilisées pour effectuer de nombreuses études. Par exemple, la plasticité des cellules souches multipotentes du tissu adipeux peut être investiguée à l'aide de cellules souches, transfectées avec le gène lacZ codant pour la β-Galactosidase (bleues après coloration Xgal). Ces cellules marquées peuvent être utilisées pour des expériences *in vitro* et *in vivo.*

Par exemple, *in vitro,* la plasticité de ces cellules peut être étudiée par des expériences de co-culture. Notamment, l'on peut analyser la capacité de ces cellules (d'origine mésodermique) à se différencier en cellules d'origine endodermique (par exemple le foie) et en cellules d'origine ectodermique (cellules nerveuses: astrocytes, oligodendrocytes et neurones).

*In vivo,* ces cellules marquées peuvent être également transplantées chez la souris athymique (nude), ayant un système immunitaire déficient et ne pouvant donc pas rejeter ces cellules. Les cellules transplantées ne donnent pas de tumeur. Le pouvoir de régénération de ces cellules n'est visible que si on effectue, au préalable, des lésions adéquates chez la souris à transplanter.

Les cellules souches de l'invention n'expriment pas de molécules HLA de classe I en surface, contrairement à la plupart des cellules somatiques. L'absence de ces protéines dont la fonction, immunitaire est cruciale, laisse supposer que ces cellules souches peuvent être transplantées universellement sans réaction de rejet.

En effet, les inventeurs ont démontrés que les cellules de l'invention peuvent être transplantées dans des souris immunocompétentes, sans réaction de rejet 6 mois après transplantation.

Les cellules CA de l'invention sont donc caractérisées en ce qu'elle présente *in vivo* un comportement immunoprivilégié, c'est-à-dire, elle ne donne pas lieu à une réaction de rejet lorsqu'elles sont transplantées dans un mammifère immunocompétent (tel qu'une souris), et ce après plus de 10 jours après la transplantation, de préférence après 80 jours, et plus préférentiellement après 6 mois. La transplantation peut être allogénique ou xénogénique. L'absence de réaction de rejet peut être évaluée à l'aide de techniques permettant de mettre en évidence l'absence d'infiltration lymphocytaire, par exemple avec des anticorps anti-CD3, ou avec une coloration à l'hématoxyline.

De façon surprenante, il a également été démontré que, *in vivo* les cellules de l'invention ont une capacité à migrer à l'état non différencié. En effet, 50 jours après transplantation de cellules CA de l'invention dans le tibialis anterior d'une souris immunocompétente, la présence de ces cellules a été constatée dans des tissus lésés adjacents au site d'injection. Ce comportement suggère que les cellules de l'invention peuvent donc contribuer, par recrutement, à la restauration d'un phénotype normal à des sites anatomiques autres que celui du site d'injection.

La présente demande décrit également des populations enrichies en cellules multipotentes, caractérisées en ce qu'elles comportent des cellules souches de l'invention, et en ce qu'elles sont dépourvues d'adipocytes, de fibroblastes, de pré-adipocytes, de cellules endothéliales, de péricytes, de mastocytes et de cellules de muscle lisse.

L'invention concerne également une population de cellules souches susceptible d'être obtenue par la mise en oeuvre du procédé selon la présente invention.

Les populations de l'invention sont de préférence entièrement homogènes, c'est-à-dire elles ne comprennent que des cellules souches. Plus particulièrement, les populations sont clonales.

L'invention concerne également la production de cellules différenciées à partir des cellules souches de l'invention.

Par exemple, l'invention concerne la production de cellules différenciées du lignage mésodermique, caractérisée en ce que l'on cultive des cellules souches de l'invention à partir de la confluence, en présence d'un milieu de différenciation approprié.

Comme milieu permettant la différenciation en adipocytes, l'on peut citer le milieu suivant :
- milieu DMEM/ Ham's F12 (vol/vol, 1:1 supplémenté d'antibiotiques par exemple 100U/ml de pénicilline, 100 µg / ml de streptomycine,
   5µg/ml insuline humaine (Sigma),
   10µg/ml de transferrine humaine (Sigma),
   Activateur PPARγ, par exemple 1µM de BRL49653, ou 2µm de Ciglitazone (Biomol),
   100 à 250 µM isobutyl-methylxanthine (IBMX)
   1 µM de dexamethasone 0.2 nM de Triiodothyronine (T3 Sigma).
   Quarante huit à soixante douze heures après, ce milieu est remplacé par le même milieu mais ne contenant plus d'IBMX et de dexamethasone.

Comme milieu permettant la différenciation en ostéoblastes, l'on peut citer le milieu suivant :
- DMEM, supplémenté d'antibiotiques par exemple 100 U/ml de pénicilline, 100 µg / ml de streptomycine,
   10% de sérum de veau décomplémenté,
   0.1 µM de dexaméthasone (SIGMA),
   10mM β-glycérophosphate (SIGMA)
   50µg/ml d'acide ascorbique (SIGMA).
   Le milieu est remplacé tous les 2-3 jours et ce pendant une période comprise entre 15 et 20 jours.

Comme milieu permettant la différenciation en myocytes, l'on peut citer les milieux suivants :
Le milieu commercialisé sous le nom de PromoCell, ou
   milieu DMEM
   2% de sérum de veau décomplémenté
   antibiotiques (par exemple 100 U/ml de pénicilline, 100 µg / ml de streptomycine)
Le milieu est remplacé tous les 2-3 jours et ce pendant 4 à 6 semaines.

Comme milieu permettant la différenciation en cellules endothéliales, l'on peut citer le milieu suivant :
Milieu DMEM supplémenté d'antibiotiques,
10 ng/ml VEGF₁₂₁ humain (SIGMA).

Pour les différenciations en adipocytes, en ostéoblastes et en myocytes, les cellules souches sont normalement ensemencées à une densité d'environ 10 000 à 25 000 cellules / cm².

Avant l'étape de différenciation, les cellules sont normalement ensemencées à une densité de 25 000 cellules / cm² dans un milieu de prolifération (DMEM supplémenté en 10% FCS et 2.5 ng/ml FGF-2). Deux jours après, le milieu de culture est changé en absence de FGF-2 pendant 48 heures. Puis les cellules sont ensuite maintenues dans un milieu de différenciation pendant 10 jours.

Les cellules souches et populations de cellules souches de l'invention sont particulièrement aptes à une utilisation en thérapie et en cosmétologie.

Les utilisations thérapeutiques des cellules souches et populations de cellules souches de l'invention comprennent, entre autres, des utilisations en transplantation et en thérapie génique.

Par exemple, pour une utilisation en transplantation, les cellules de l'invention sont multipliées à l'état indifférencié *in vitro,* suivi de l'introduction des cellules chez un individu. Les cellules peuvent être soit injectées dans la circulation, soit implantées dans un site anatomiques. Les cellules se différencient alors *in vivo* en fonction du site anatomique lésé. Par exemple, la transplantation intramusculaire de cellules souches de l'invention chez un individu présentant des lésions musculaires, donnera lieu à une différenciation et régénération du muscle. De même, la régénération de tissu adipeux peut être envisagée par différenciation *in vivo* des cellules en adipocytes.

La transplantation des cellules de l'invention peut donc être utilisée pour la régénération de tissu *in vivo,* par exemple un tissu osseux, un tissu adipeux, ou un tissu musculaire.

Le cas échéant, la transplantation peut être accompagnée de l'implantation d'une matrice susceptible d'améliorer la régénération du tissu, par exemple en fournissant un support physique pour la prolifération des cellules ou en fournissant des substances telles que des facteurs de croissance etc. La matrice peut être bio-dégradable.

Selon l'invention, la transplantation peut être autologue ou allogénique. Les cellules de l'invention sont particulièrement adaptées aux allo-transplantations en raison de leur caractère HLA Classe I négatif. Les cellules peuvent donc être utilisées chez tout individu indépendamment de son génotype, sans risque de rejet.

Selon une autre variante, les cellules souches de l'invention peuvent être utilisées à l'état différencié, par exemple en adipocytes, en chondrocytes, en ostéoblastes, en myocytes etc. Selon cette variante de l'invention les cellules souches sont soumises à une différenciation *in vitro,* suivie par l'introduction des cellules différenciées chez un individu.

Pour les applications thérapeutiques de l'invention les cellules souches peuvent être génétiquement modifiées ou non. Lorsque les cellules sont génétiquement modifiées, elles peuvent être utilisées en thérapie génique pour apporter un produit d'expression chez un patient, par exemple une protéine hétérologue. Les cellules modifiées peuvent être cultivées in vitro à l'état indifférencié puis introduites chez le récipient. Alternativement, les cellules peuvent être multipliées in vitro à l'état différencié et ensuite introduites chez le récipient.

L'invention concerne également la mise en oeuvre de méthodes chirurgicales et thérapeutiques employant les cellules souches de l'invention. Elle concerne également les compositions pharmaceutiques comprenant les cellules souches de l'invention ou une population de cellules souches selon l'invention en association avec un excipient physiologiquement acceptable.

Les cellules souches de l'invention peuvent également être utilisées pour la production *in vitro,* de protéines, recombinantes ou non, particulièrement des protéines thérapeutiques. En effet, les cellules de l'invention peuvent être cultivées *in vitro* pendant au moins 100, par exemple au moins 200 doublements de population, et constituent donc une source quasiment inépuisable de produits d'expression. Les protéines en question peuvent être des produits d'expression de gènes endogènes aux cellules souches, ou alternativement, peuvent être des produits d'expression de gènes hétérologues.

Les cellules ou populations de cellules de l'invention peuvent également être utilisées dans des systèmes de criblage pour identifier des agents actifs, par exemple des produits de gènes, des extraits sériques, des milieux conditionnés, des produits d'origine animale ou végétale, des banques d'agents pharmacologiques etc.

Par exemple, l'invention comprend un procédé de criblage permettant d'identifier des agents susceptibles de moduler la différenciation de cellules en cellules du lignage mésodermique, caractérisé par :
a) la mise en culture de cellules souches ou d'une population de cellules souches selon l'invention dans des conditions permettant leur différenciation en cellules du lignage mésodermique, (par exemple en adipocytes, en ostéoblastes ou en myocytes) en présence d'un agent candidat,
b) comparaison de la différenciation des cellules en présence de l'agent candidat avec la différenciation en l'absence de l'agent candidat.

L'agent sous test peut être un agent susceptible d'augmenter la différenciation, ou un agent susceptible d'empêcher ou ralentir ou diminuer la différenciation (substance anti-différenciatrice), ou encore une substance susceptible de modifier la voie de différenciation.

L'invention comprend également un procédé de criblage permettant d'identifier des agents susceptibles de présenter une activité lipolytique, caractérisé par :
a) la mise en culture de cellules souches ou d'une population de cellules souches selon l'invention dans des conditions permettant leur différenciation en adipocytes,
b) mise en contact des adipocytes ainsi obtenus, avec un agent candidat, évaluation de l'activité lipolytique de l'agent candidat.

L'invention comprend également un procédé de criblage permettant d'identifier des agents susceptibles de présenter une activité anti-lipolytique, caractérisé par :
a) la mise en culture de cellules souches ou d'une population de cellules souches selon l'invention dans des conditions permettant leur différenciation en adipocytes,
b) mise en contact des adipocytes ainsi obtenus, avec un agent candidat, en présence d'un agent lipolytique,
c) évaluation de l'activité anti-lipolytique de l'agent candidat.

L'invention comprend également un procédé de criblage permettant d'identifier des agents susceptibles de présenter une activité insulino-sensibilisante, caractérisé par :
a) la mise en culture de cellules souches ou d'une population de cellules souches selon l'invention dans des conditions permettant leur différenciation en adipocytes,
b) mise en contact des adipocytes ainsi obtenus, avec un agent candidat,
c) évaluation de l'activité insulino-sensibilisante de l'agent candidat, comparé à des adipocytes non-traités.

L'invention porte également sur l'utilisation des cellules souches ou d'une population de cellules souches selon l'invention dans la cosmétique.

Dans la mesure où les cellules souches de l'invention peuvent se différencier en adipocytes, elles peuvent être utilisées en chirurgie esthétique ou réparatrice, par exemple pour réduire l'aspect ridé de la peau, pour atténuer les cicatrices ou marques cutanées diverses, pour effectuer un remplissage tissulaire. L'invention concerne donc la mise en oeuvre de ces méthodes chirurgicales employant les cellules de l'invention.

Les cellules souches ou populations de cellules souches selon l'invention peuvent aussi être incluses dans des compositions cosmétiques comprenant des excipients, véhicules, solvants, colorants, parfums, antibiotiques ou autres produits et additifs habituellement utilisés dans les produits cosmétiques. L'inclusion des cellules dans des crèmes, pommades, onguents, gels, fluides divers etc, permet leur application directement sur la peau ou autres tissus ou phanères. L'invention porte donc également sur les compositions cosmétiques contenant les cellules souches de l'invention à l'état non-différencié, ou contenant des cellules différenciées dérivées des cellules souches.

### Légendes des Figures

Différents aspects de l'invention sont illustrés dans les figures :

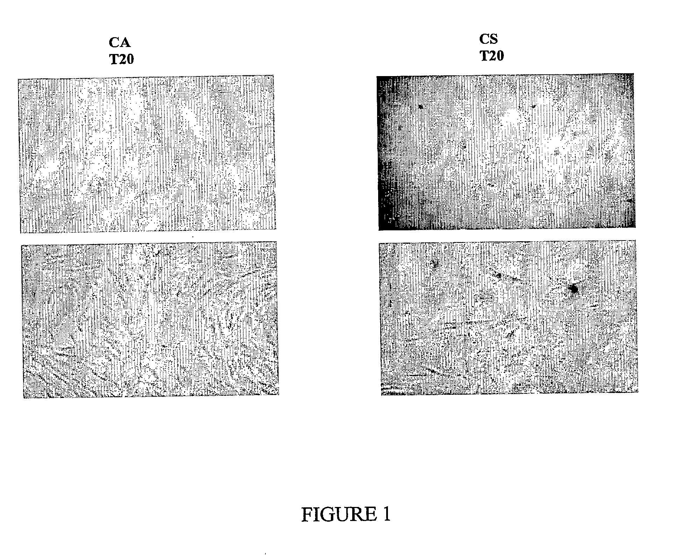

Figure 1 : **Activité** β**-galactosidase endogène des cellules CA et CS détectée à pH 6.**
   Une coloration Xgal, révélant l'activité β-galactosidase endogène (traduisant la sénescence des cellules), effectuée au stade de 60 doublements de population, correspondant au transfert cellulaire 20 (« T20 »), révèle que la population CS est sénescente (Taux de sénescence 0.415 ± 0.025%), alors que la population CA est simplement quiescente (Taux de sénescence 0.045 ± 0.01 %).

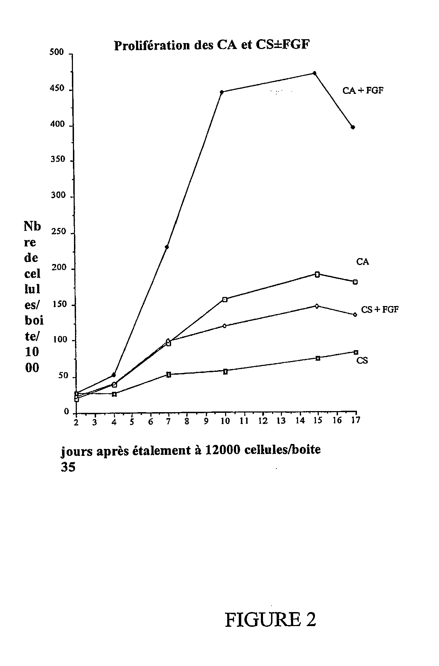

Figure 2 : **Effet du bFGF (basic Fibroblast Growth Factor) à la concentration de 5 ng/ml de milieu) sur les populations CA et CS, à des stades tardifs, c'est à dire après 50 doublements de population.**
   La Figure 2 montre la prolifération des CA ± bFGF et CS ± bFGF (Abscisse : jours après étalement à 12 000 cellules / boîte de 35 mm de diamètre ; Ordonnée : nombre de cellules (x 1000) / boîte). Seules les cellules de la population CA répondent de manière efficace au bFGF. Après 50 doublements de population, le bFGF n'a pas d'effet significatif sur la population CS. Ces observations confirment l'état de quiescence de la population CA et l'état de sénescence de la population CS.

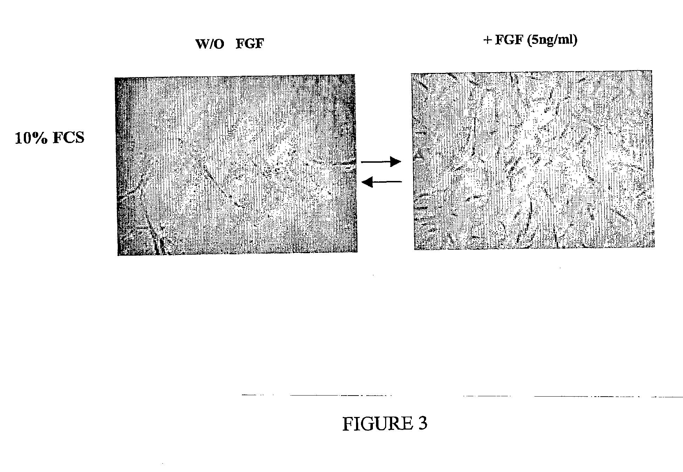

Figure 3 : **Morphologie des cellules CA à des stades tardifs (après 50 doublements de population) en l'absence de bFGF et en présence de bFGF.**
   « W/O FGF » = en l'absence de bFGF
   « +FGF » = en présence de bFGF (5 ng/ml)
   « FCS » = sérum de veau foetal («Foetal Calf Sérum »)

   Le bFGF entraîne un changement de la morphologie des cellules. Quiescentes, celles-ci sont aplaties et élargies. En présence de bFGF, et donc en phase proliférative, elles prennent une forme fibroblastique.
   L'effet du bFGF est réversible.

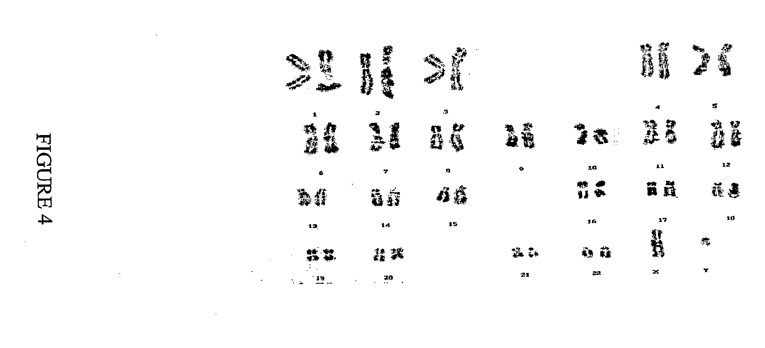

Figure 4: **Caryotype des cellules de la Primo 2 CA.**
   Le caryotype des cellules de la primo 2 a été réalisé sur les 2 sous populations CA et CS, en présence ou non de bFGF et à différents passages. Pour les cellules de la Primo 2CA les caryotypes ont été réalisés aux stades suivants : T21 = 80 doublements de population ; T23 = 90 doublements de population ; T34 = 130 doublements de population. Dans tous les cas, les caryotypes sont normaux. La Figure 4 montre un exemple de caryotype des cellules de la primo 2CA.
Figure 5 : **Différenciation in vitro des cellules de la primo 2 CA et de la Primo 2 CS en adipocytes** à des passages précoces :
   CST1 : population CS au passage 1 (correspondant à 3 doublements de population) ;
   CST7 : population CS au passage 7 (correspondant à 21 doublements de population) ;
   CAT5 : population CA au passage 5 (correspondant à 15 doublements de population). Coloration Oil Red O.
Figure 6: **Expression transcriptionnelle de marqueurs adipogéniques au cours de la différenciation de cellules Primo 2**. Analyse en Northern Blot.
   J0, J7, J12 = 0, 7 et 12 jours, respectivement, après induction de la différenciation,
   CA T14 : population Primo 2 CA au passage 14 (correspondant à 42 doublements de population).
   CS T16 : population Primo 2 CS au passage 16 (correspondant à 48 doublements de population) ;
Figure 7 : **Différenciation in vitro en ostéoblastes de cellules CA et CS à des passages précoces.**
   CST1 : population CS au passage 1 (correspondant à 3 doublements de population) ;
   CST7 : population CS au passage 7 (correspondant à 21 doublements de population) ;
   CAT5 : population CA au passage 5 (correspondant à 15 doublements de population). Coloration Alizarin Red.
Figure 8 : **Capacité de différenciation de cellules CA à des passages tardifs.**
   A : Différenciation adipocytaire de cellules de la Primo 2CA. L'induction de différenciation adipocytaire est effectuée lorsque les cellules sont au stade T30 (environ 130 doublements de population). Coloration Oil Red O.
   B : Différenciation ostéoblastique de cellules de la Primo 2CA. L'induction de différenciation ostéoblastique est effectuée lorsque les cellules sont au stade T30 (environ 130 doublements de population. Coloration Alizarin Red.

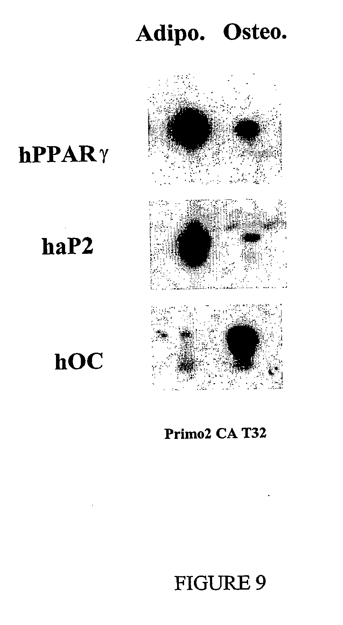

Figure 9 : **Fonctionnalité des adipocytes et des ostéoblastes : Expression transcriptionnelle de marqueurs spécifiques soit de la différenciation adipocytaire, soit de la différenciation ostéoblastique.**
   L'expression transcriptionnelle des marqueurs hPPARγ-2, haP2 et hOC est déterminée par RT-PCR. Les cellules utilisées sont i) des adipocytes (côté gauche de la Figure), issues de Primo2CA, l'induction adipocytaire ayant eu lieu au stade T32 (environ 140 doublements de population), et ii) des ostéoblastes (côté droite de la Figure), issues de Primo2CA, l'induction ostéoblastique ayant eu lieu au stade T32 (environ 140 doublements de population).
   hPPARγ-2 : récepteur γ humain activé par les proliférateurs de péroxisome ("human peroxisome proliferator activated receptor y") : marqueur adipocytaire.
   haP2 : protéine de liaison des acides gras humains ("human fatty acid binding protein") : marqueur adipocytaire,
   hOC : ostéocalcine humaine (marqueur ostéoblastique)

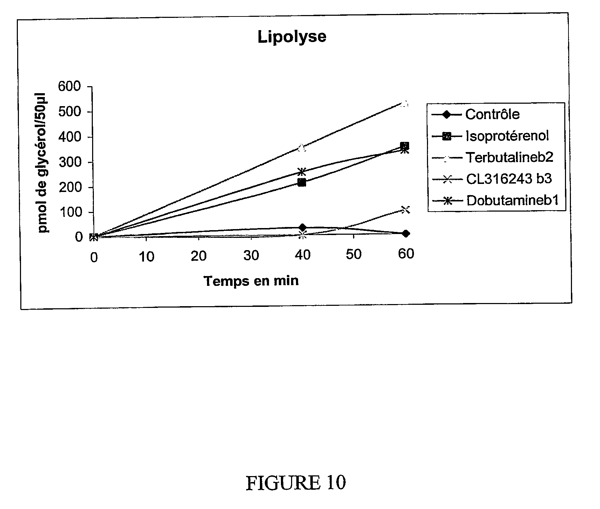

Figure 10 : **Fonctionnalité des adipocytes : Capacité de lipolyse des cellules de la Primo 2CA.**
   Des lipolyses ont été réalisées sur des adipocytes obtenus à partir de cellules Primo2CA T32 (environ 140 doublements de population), avec des agonistes spécifiques de différents récepteurs β-adrénergiques. La Figure 10 montre les vitesses de lipolyse obtenues, et confirment l'absence de récepteurs β3 adrénergiques.

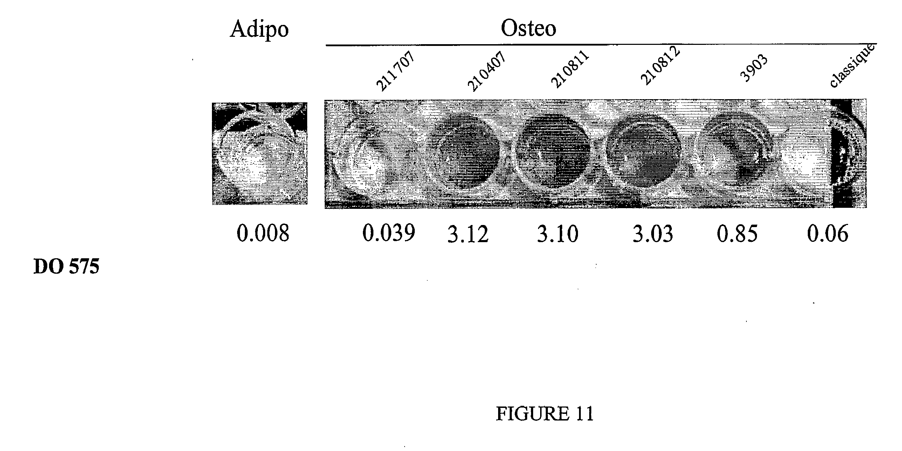

Figure 11 : **Fonctionnalité des ostéoblastes : Détection de calcium associé à la matrice extracellulaire.**
   La matrice extracellulaire présente dans les boîtes de culture après lyse du tapis cellulaire, est séchée puis incubée avec la solution du "SIGMA calcium detection kit". La quantité de calcium sécrété par les ostéoblastes est quantifiée par lecture de cette solution au spectrophotomètre (DO 575). La fonctionnalité des ostéoblastes se confirme.
   La quantité de calcium sécrétée par les ostéoblastes varie en fonction du lot de sérum (211707, 210407, 210811, 210812, 3903, classique). Ceux-ci peuvent contenir des cytokines, hormones ou facteurs de croissance non caractérisés et présents dans des proportions variables.
   Les adipocytes ne sécrètent pas de quantité significative de calcium.

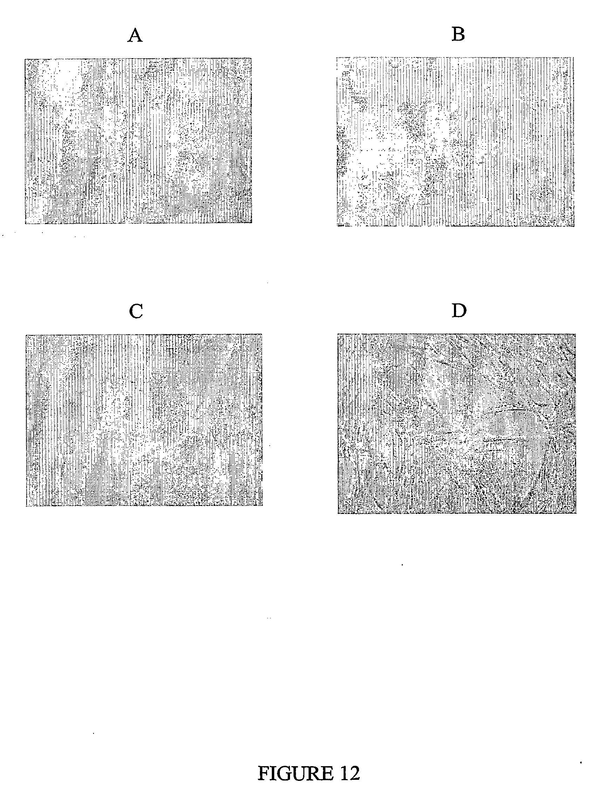

Figure 12 : **Morphologie des cellules de la Primo2CA en fonction du nombre de doublements de population**
   A : 40 doublements de population
   B : 100 doublements de population : quiescentes
   C : 150 doublements de population : quiescentes
   D : 150 doublements de population + bFGF : en phase proliférative.

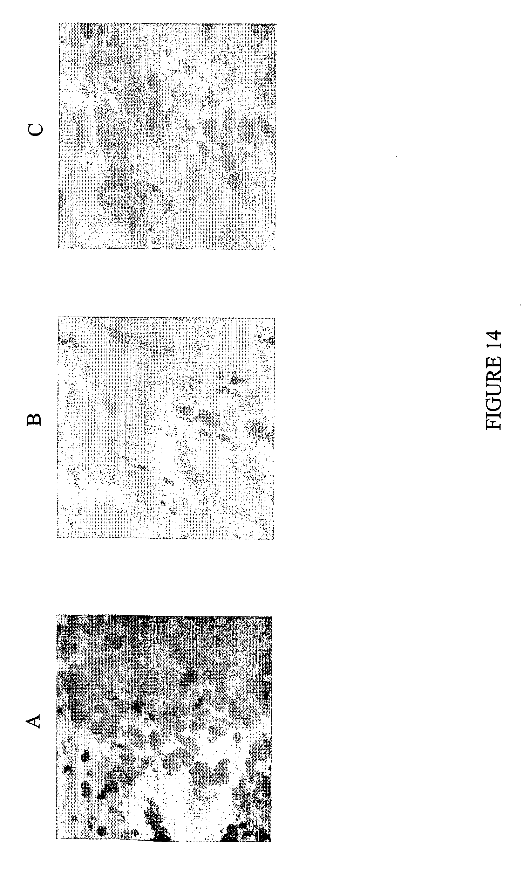

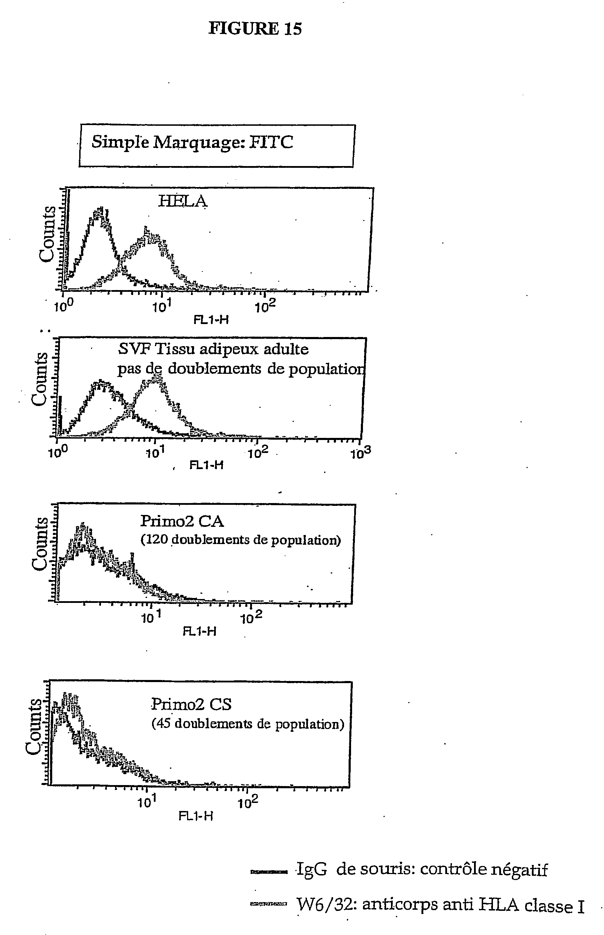

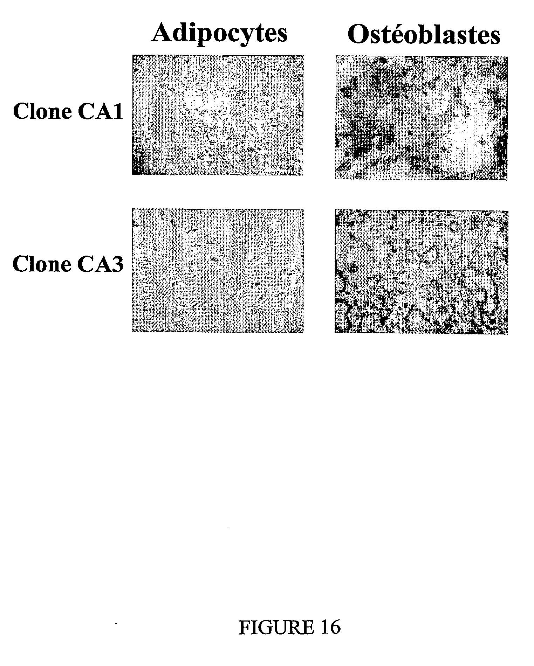

   Le bFGF entraîne un changement de la morphologie des cellules. Quiescentes, celles-ci sont aplaties et élargies. En présence de bFGF, et donc en phase proliférative, elles prennent une forme fibroblastique (voir aussi Figure 3).

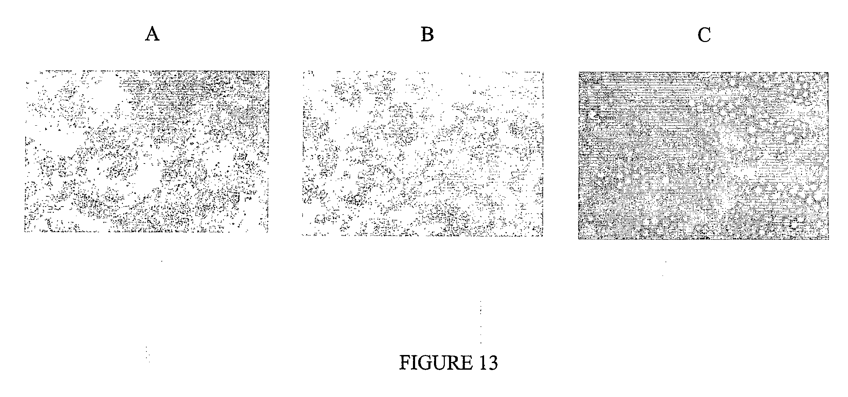

Figure 13 : **Capacité de différenciation adipocytaire des Primo 1CA, Primo 3CA et Primo 6CA:** Adipocytes après 8 jours de différenciation.
   A : Primo 1 : différenciation induite à 50 doublements de population
   B : Primo 3 : différenciation induite à 40 doublements de population
   C : Primo 6 : différenciation induite à 40 doublements de population
Figure 14: **Capacité de différenciation adipocytaire des Primo 1CA, Primo 3CA et Primo 6CA : Coloration Oil red O**
   A : Primo 1 : différenciation induite à 40 doublements de population
   B : Primo 3 : différenciation induite à 25 doublements de population
   C : Primo 6 : différenciation induite à 25 doublements de population
Figure 15 : **Marquage cellulaire et analyse en cytométrie de Flux.**
   Mise en évidence du caractère HLA Classe I négatif des cellules souches Primo 2CA.
   *Simple Marquage: FITC*
      1. HELA : Cellules humaines tumorales. HLA Classe I positif.
      2. SVF : Tissu adipeux adulte, pas de doublements de populations. HLA Classe I positif
      3. Primo 2CA : 120 doublements de populations
      4. Primo 2CS : 45 doublements de populations
   Courbe noire : IgG de souris : contrôle anticorps négatif
   Courbe grise : W6/32 Anticorps anti-HLA Classe I
Figure 16 : **Obtention de clones multipotents à partir de cellules CA.**
   Les clones CA1 et CA3 ont été mis dans le milieu de culture permettant la différenciation en adipocytes et en ostéoblastes. Les adipocytes sont révélés par le colorant à l'huile rouge et les ostéoblastes par le rouge Alizarin.

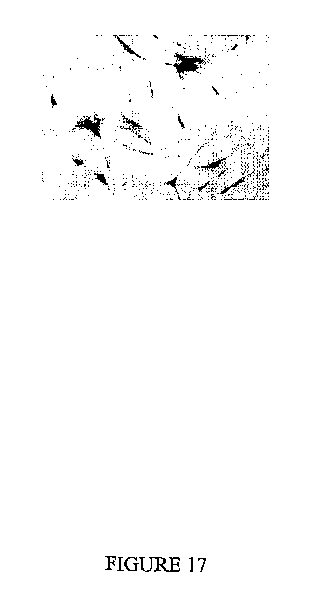

Figure 17 : **Expression d'un transgène dans les cellules souches CA.**
   Les cellules souches CA sont transduites par un rétrovirus exprimant un gène de résistance à un antibiotique, la puromycine et le gène rapporteur LacZ. Elles sont ensuite sélectionnées en présence de puromycine. Les cellules sélectionnées expriment toutes le gène LacZ révélé in situ par l'activité β-galactosidase.

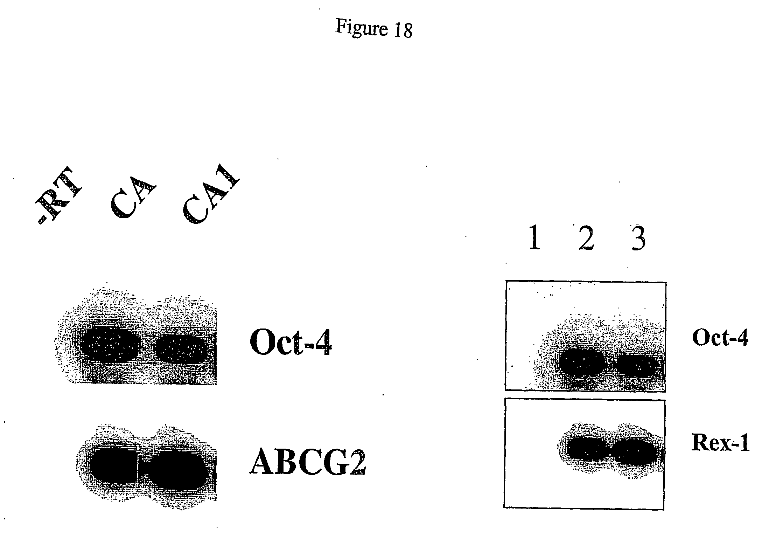

Figure 18 : **Expression de Oct-4, de Rex-1 et d'ABCG2 dans les cellules souches CA :**
   *Photos de gauche :* Expression de l'ARN Oct-4 et ABCG2 dans les cellules CA et le clone CA1
   Les ARN sont extraits à partir des cellules CA et CA1 et l'expression de Oct-4 et ABCG2 est :
   - amplifiée par RT-PCR
   - puis détectée par hybridation.

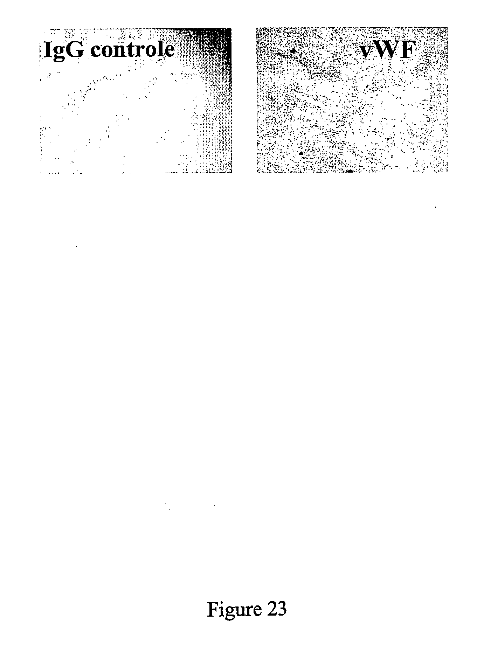

   *Photos de droite :* Expression du facteur de transcription Rex-1 par des cellules primo 2CA après 80 et 160 doublements de population. Le facteur de transcription Rex-1 est impliqué dans le maintien à l'état non différencié de cellules souches embryonnaires. Les chiffres « 1 », « 2 » et « 3 » signifient « -RT (contrôle négatif) », « cellules CA », et « cellules CA1 », respectivement.
   Les conditions de PCR sont :
   - pour Oct-4 : 94°C, 1 mn; 57°C, 1 mn; 72°C, 1 mn pendant 45 cycles.
      Amorces : 5'-GACAACAATGAAAATCTTCAGGAGA-3' et
      5'-TTCTGGCGCCGGTTACAGAACCA-3',
      amorce interne 5'-CACTCGGTTCTCGATACTGG-3' pour un fragment de 220-pb.
   - pour ABCG2 : 94°C, 1mn; 60°C, 1mn; 72°C, 1mn pendant 31 cycles,
      Amorces : 5'-GGCCTCAGGAAGACTTATGT-3' et
      5'-AAGGAGGTGGTGTAGCTGAT-3'
   - pour Rex-1 : 94°C, 1min, 60°C 1 min, 72°C 1min, Nombre de cycles 31 ; 72° 5min, 1 cycle
      Amorce : 5'-CTCTCCAGTATGAACCAGG-3' et
      5'-GAAAGGATCAGAACAACAGC-3',
      amorce interne, 5'-GGCATTGACCTATCAGATCC-3' pour un fragment de 400-pb.
Figure 19: **Représentation graphique d'une variante préférée du procédé pour la production de cellules souches adultes à partir de tissu adipeux humain.**

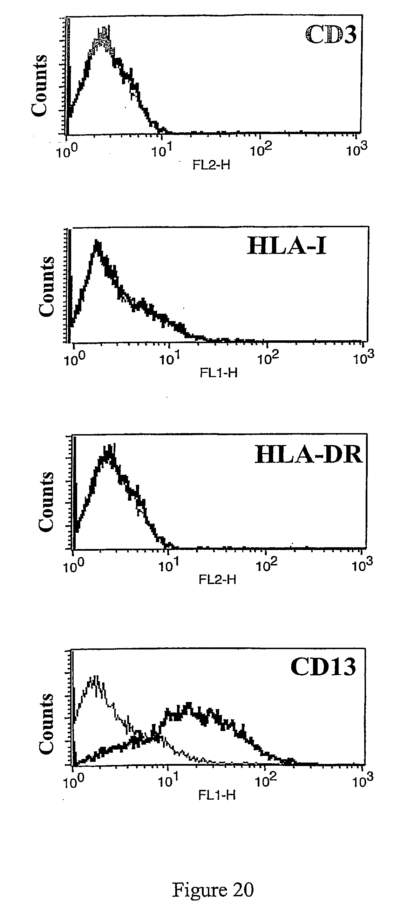

Figure 20 : **Caractéristiques des cellules primo2 CA en termes de marqueurs de surface.**
   Des cellules primo2 CA au stade 80, 120 et 160 doublements de population ont été marquées avec des anticorps anti-HLA I, anti-HLA-DR anti-CD3, anti-CD13 couplés au préalable avec le FITC ou la phycoérythrine. Contrôle = IgG, les anticorps utilisés sont les suivants : HLA de classe I conjugué à la fluorescéine (FITC) ; HLA-DR (HLA classe II) conjugué à la phycoérythrine (PE) ; CD3 (marqueur des lymphocytes T) conjugué à PE ; CD13 (marqueurs des cellules stromales de la moelle osseuse, cellules endothéliales, progéniteurs précoces des granulocytes/monocytes et de leur descendance) conjugué au FITC.
   Ligne fine : IgG contrôle
   Ligne grasse : anticorps d'intérêt.

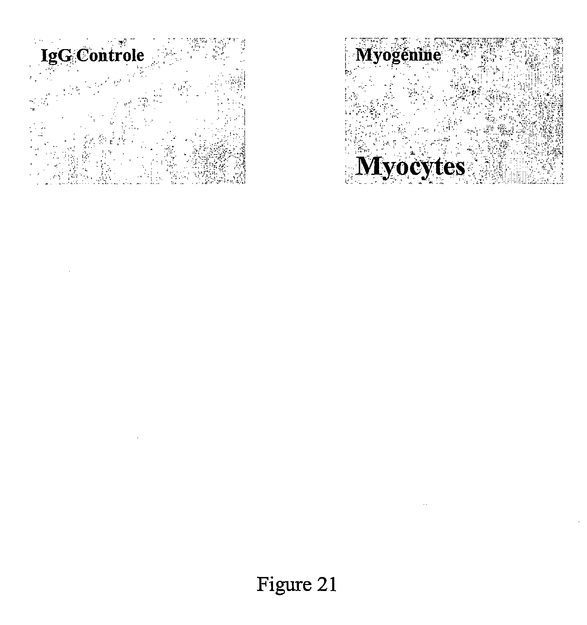

Figure 21 : **Différenciation myocytaire in-vitro après 4 jours.**
   Détection par immuno-histochimie de la myogénine, facteur précoce de la différentiation myocytaire. La figure 21 illustre des micrographes de cellules primo2 CA à 150 doublements de population, après 4 jours en présence de milieu de différentiation en myocytes. Les cellules sont fixées avec 4% de paraformaldéhyde pendant 10 minutes à température ambiante et perméabilisées en présence de PBS/0.1% triton X100 pendant 10 minutes ; on procède ensuite au blocage de l'activité péroxydase endogène en incubant les cellules avec 3% de H2O2 pendant 5 minutes. Les cellules sont ensuite incubées avec l'anticorps primaire : anticorps anti-myogénine (mouse anti-human IgG) (1 :100) entre 30 minutes et une heure à température ambiante puis avec l'anticorps secondaire (anti-mouse IgG couplé à la péroxydase).

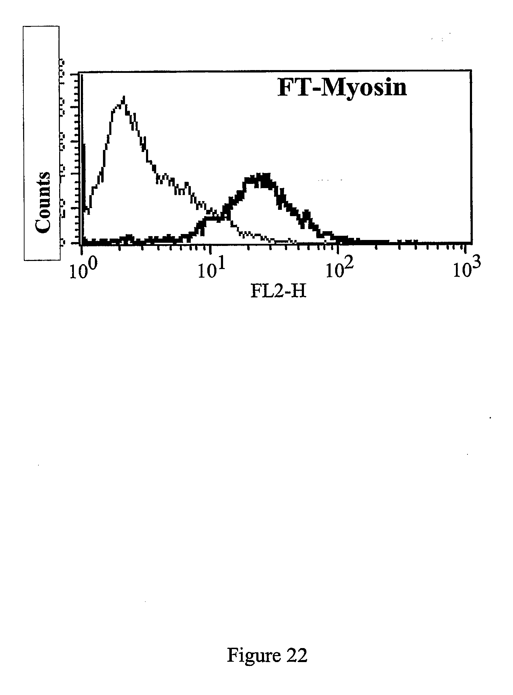

Figure 22 : **Différenciation myocytaire in-vitro après 21 jours.**
   Après 21 jours en présence de milieu de différentiation en myocytes, détection de la myosine des fibres musculaires rapides (« fast twitch myosin » ou « FT myosin »), marqueur tardif de la différentiation myocytaire (marqueur intra-cellulaire). Détection par FACS : après décollement des cellules primo2 CA (150 doublements de population) celles-ci sont fixées en présence de PBS/ 1% formaldéhyde pendant 15 minutes à température ambiante puis perméabilisées avec une solution de digitonine (10µg/ml de PBS) pendant 7 à 8 minutes à température ambiante. On procède ensuite au marquage anticorps en suivant le protocole décrit pour l'expression des marqueurs de surface (figure 20). L'anticorps utilisé est un anticorps de souris conjugué directement à la phycoérythrine et reconnaissant la FT-myosine humaine.
   Ligne fine : IgG contrôle
   Ligne grasse : FT-myosine
Figure 23 : **Différenciation en cellules endothéliales in vitro**
   Détection par immuno-histochimie du « von willebrandt factor » (vWF), marqueur spécifique des cellules endothéliales. La figure 23 illustre l'expression du vWF par les cellules primo2 CA à 150 doublements de population, après 21 jours en présence de milieu angiogénique. Les cellules sont fixées avec 4% de paraformaldéhyde pendant 10 minutes à température ambiante et perméabilisées en présence de PBS/0.1% triton X100 pendant 10 minutes ; on procède ensuite au blocage de l'activité péroxydase endogène en incubant les cellules avec 3% de H2O2 pendant 5 minutes. Les cellules sont ensuite incubées avec l'anticorps primaire : anti-vWF (Goat IgG reconnaissant à la fois le vWF humain, de rat et de souris) entre 30 minutes et 1 heure à température ambiante puis avec l'anticorps secondaire, anti-goat-IgG couplé à la péroxydase (1 :100). Après 21 jours de maintien dans un milieu composé de DMEM et de hVEGF121 (10ng/mL), les cellules de la primo 2CA expriment le vWF.

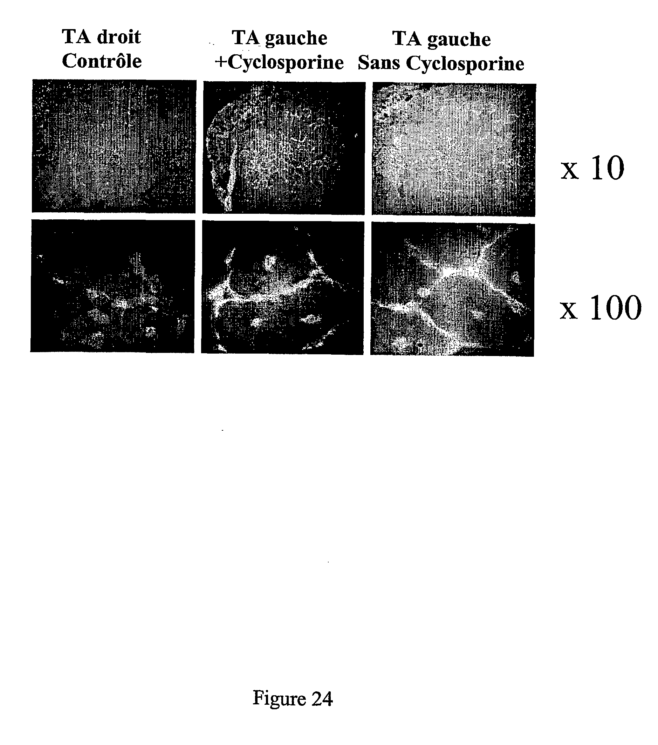

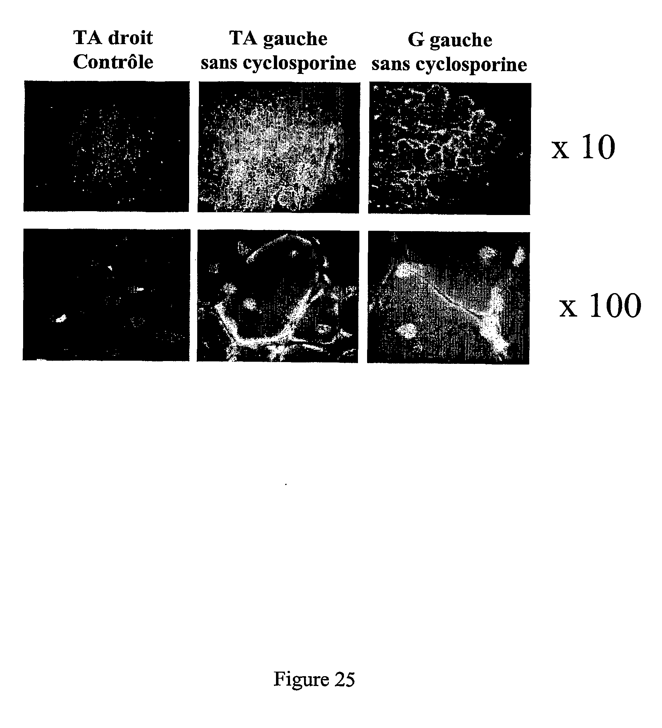

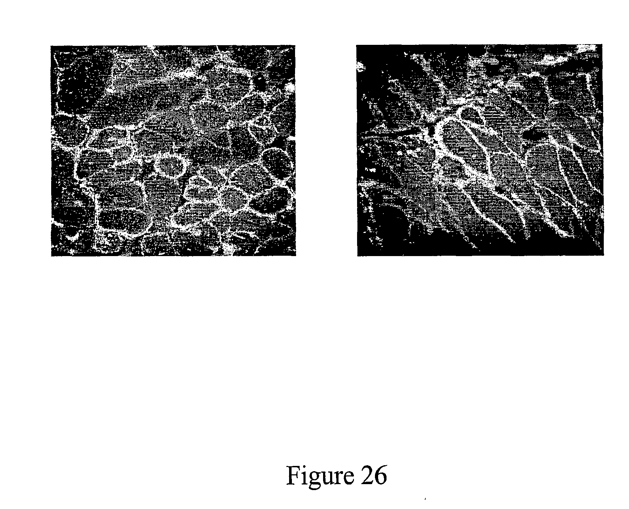

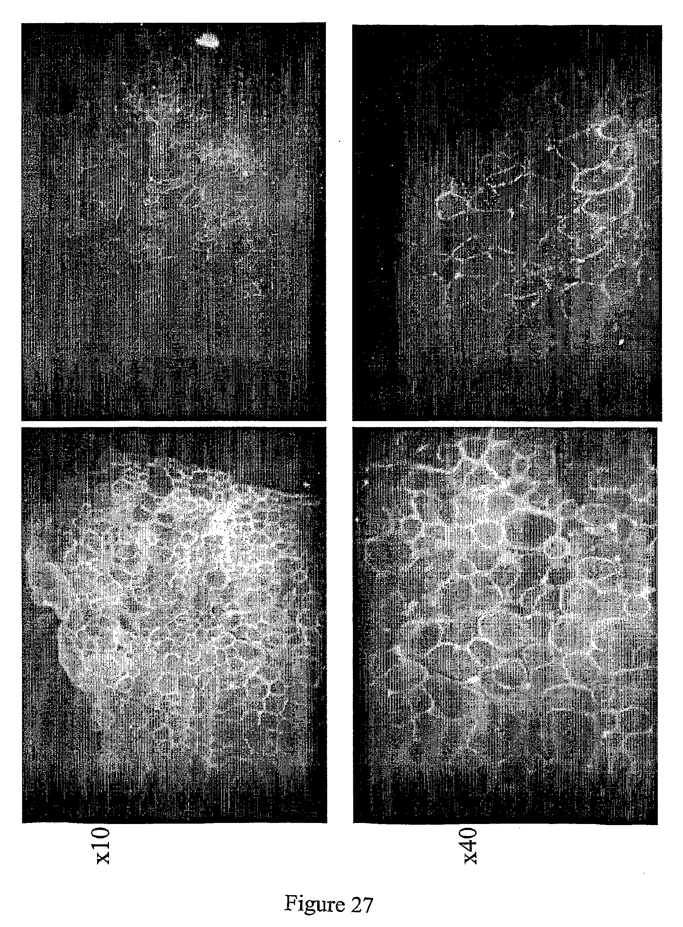

Figures 24 à 27 : **Pouvoir de régénération musculaire in vivo des cellules de la primo 2 CA après 10 jours, 50 jours, 80 jours et 6 mois de transplantation chez la souris mdx sans immunosuppresseur**: Les Figures 24, 25, 26 et 27 illustrent la co-localisation, après 10 jours (Fig 24), 50 jours (Fig. 25), 80 jours (Fig. 26) et 6 mois (Fig. 27) de transplantation de cellules Primo 2CA dans le Tibialis Anterior, de noyaux humains avec les fibres musculaires re-exprimant la dystrophine. Cette co-localisation a été effectuée par double marquage de la dystrophine par immunofluorescence et des noyaux humains par FISH. Les cellules transplantées sont des cellules Primo 2CA à 160 doublements de populations, au nombre de 150000.

L'étape de détection de la dystrophine par immunofluorescence a été effectuée avant de procéder au marquage des noyaux humains par FISH :
- *Détection de la dystrophine par immunofluorescence :* les coupes de muscles sont incubées pendant une heure avec un anticorps reconnaissant la dystrophine humaine, préalablement couplé à la fluorescéine. Les anticorps utilisés sont soit :
un anticorps spécifique de la dystrophine humaine et souris (mouse anti-human IgG1 : NCL-DYS2 de Novocastra, dirigé contre l'extrémité C-terminale de la dystrophine humaine et souris) soit,
un anticorps spécifique de la dystrophine humaine (mouse anti-human IgG2a : NCL-DYS3 de Novocastra, dirigé contre l'extrémité N-terminale de la dystrophine humaine)

- *Détection des noyaux humains par FISH :* La sonde utilisée pour détecter les noyaux humains est une sonde spécifique de tous les centromères humains (α-Satellite) couplée à la digoxigénine (CP5095-DG.5, Appligene Oncor). L'étape de détection consiste en l'application sur les lames d'un anticorps anti-digoxigénine couplé à la rhodamine. Avant l'analyse des coupes, on procède à la coloration de l'intégralité des noyaux en utilisant une solution de DAPI (coloration bleue). Les lames sont ensuite observées au microscope à fluorescence (Axiophot Zeiss) avec une lampe 100 watts et un système de filtres (Perceptive Scientific International).

| | |
|---|---|
| En vert : | dystrophine ; |
| En rouge : | centromères humains |
| En bleu : | noyaux (humains et murins) |
| TA: | Tibialis Anterior |
| G: | Gastrocnémius |

| | |
|---|---|
| **Fig 24** **(10 jours) :** | anticorps anti-dystrophine : NCL-DYS2 |
| | photos de gauche : Tibialis Anterior non-traité (contrôle) ; |
| | photos du centre : Tibialis Anterior d'une souris mdx traitée avec de la cyclosporine, **10** jours après transplantation ; |
| | photos de droite : Tibialis Anterior d'une souris mdx immunocompétente (sans cyclosporine), **10** jours après transplantation ; |
| **Fig 25** **(50 jours) :** | anticorps anti-dystrophine : NCL-DYS2 |
| | photos de gauche : Tibialis Anterior non-traité (contrôle) ; |
| | photos du centre : Tibialis Anterior d'une souris mdx immunocompétente, 50 jours après transplantation ; |
| | photos de droite : Gastrocnémius d'une souris mdx immunocompétente (sans cyclosporine), **50** jours après transplantation, dans le TA adjacent. |
| **Fig 26** **(80 jours) :** | anticorps anti-dystrophine : NCL-DYS2 |
| | photo de gauche : Tibialis Anterior d'une souris mdx immunocompétente (sans cyclosporine), **80** jours après transplantation ; |
| | photo de droite : Gastrocnémius d'une souris mdx, immunocompétente (sans cyclosporine) **80** jours après transplantation, dans le TA adjacent. |
| **Fig 27** **(6 mois) :** | anticorps anti-dystrophine : NCL-DYS3 |
| | photos de gauche : Tibialis Anterior d'une souris mdx immunocompétente (sans cyclosporine), **6 mois** après transplantation ; |
| | photos de droite : Tibialis Anterior d'une souris mdx non-traitée (témoin). |

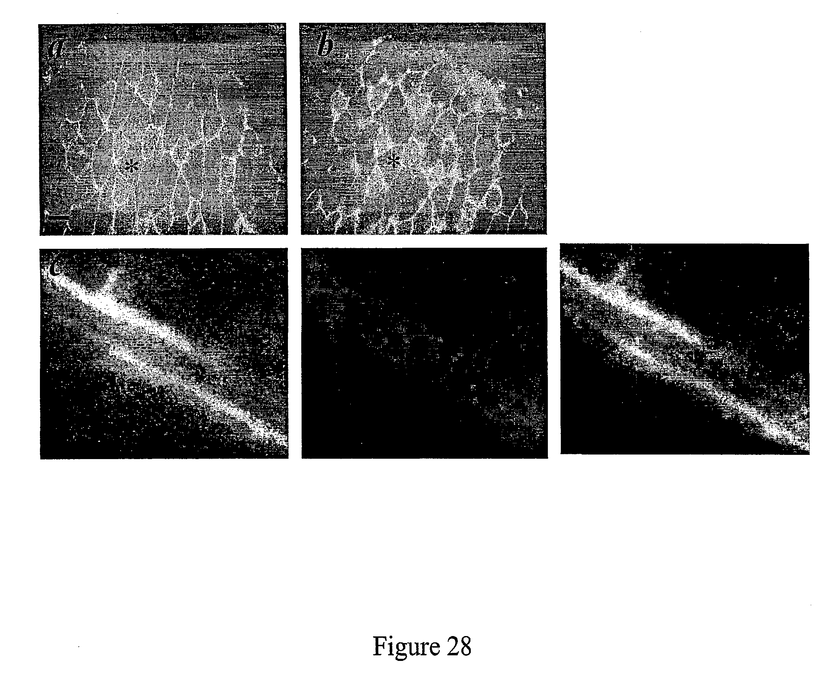

Figure 28 : **Mise en évidence, par immunodétection comparative, de l'origine humaine de la dystrophine exprimée dans les myofibres du muscle transplanté :** L'analyse de la présence de myofibres exprimant de la dystrophine, et la localisation subcellulaire dans le tibialis anterior 10 jours après transplantation, a été effectuée en utilisant les anticorps suivants :
   (a) : un anticorps dirigé contre l'extrémité C-terminale de la dystrophine humaine et souris (mouse anti-human IgG1 : NCL-DYS2 de Novocastra,),
   (b) et (c) : un anticorps dirigé contre l'extrémité N-terminale de la dystrophine humaine (mouse anti-human IgG2a : NCL-DYS3 de Novocastra,),
   (d) et (e) : un anticorps spécifique de la collagène III de souris.

   La barre indiquant l'échelle correspond à 15 µm dans les Figures 28(a) et 28(b), et à 1 µm dans les figures 28(c) à (e). L'étoile * indique une section d'un même myofibre.
   La similarité entre les Figures 28(a) et (b) indique l'origine humaine de la dystrophine exprimée. La dystrophine humaine est localisée sous le sarcolemne. En revanche, la collagène III de souris est présente dans l'espace extracellulaire entre les myofibres (figures 28(c) à (e)).

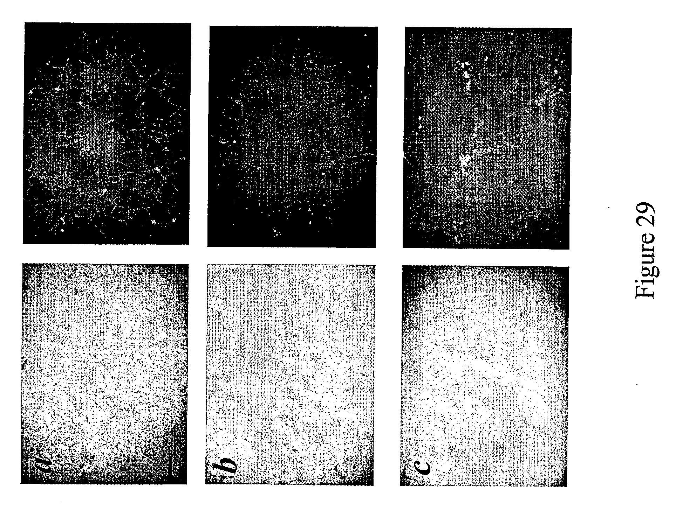

Figure 29 : **Absence de réactions immunitaires cellulaires et humorales 10 jours après transplantation de cellules souches de l'invention :** L'existence d'une éventuelle infiltration lymphocytaire à la suite de la transplantation de cellules Primo 2CA chez une souris mdx immunocompétente a été étudiée à l'aide de l'hématoxyline (Figs 29(a), (b) et (c)), ou d'anticorps anti-CD3 de souris (Figs 29(a'), (b') et (c')).
   Fig 29(a) et (a') : Tibialis anterior de souris mdx immunocompétente non traitée (témoin) ;
   Fig 29 (b) et (b') : Tibialis anterior de souris, **10 jours** après transplantation de cellules Primo 2CA, au nombre de 150,000 ;
   Fig.29 (c) et (c') : Tibialis anterior de souris, **10 jours** après transplantation de cellules stromales-vasculaires humaines non purifiées, isolées à partir de tissu adipeux ;

   La barre indiquant l'échelle correspond à 50 µm dans les Figures 29(a) à (c), et à 20 µm dans les Figures 29(a') à (c').
   10 jours après transplantation de cellules Primo 2CA, aucune infiltration lymphocytaire (CD3⁺) n'est observée (voir Fig. 29(b) et (b'), comparées aux Figures 29 (a) et (a')). En revanche, la transplantation de cellules stromales-vasculaires non purifiées, isolées à partir de tissu adipeux humain induit une réaction immunitaire cytotoxique et humorale (Fig. 29(c) et (c')).

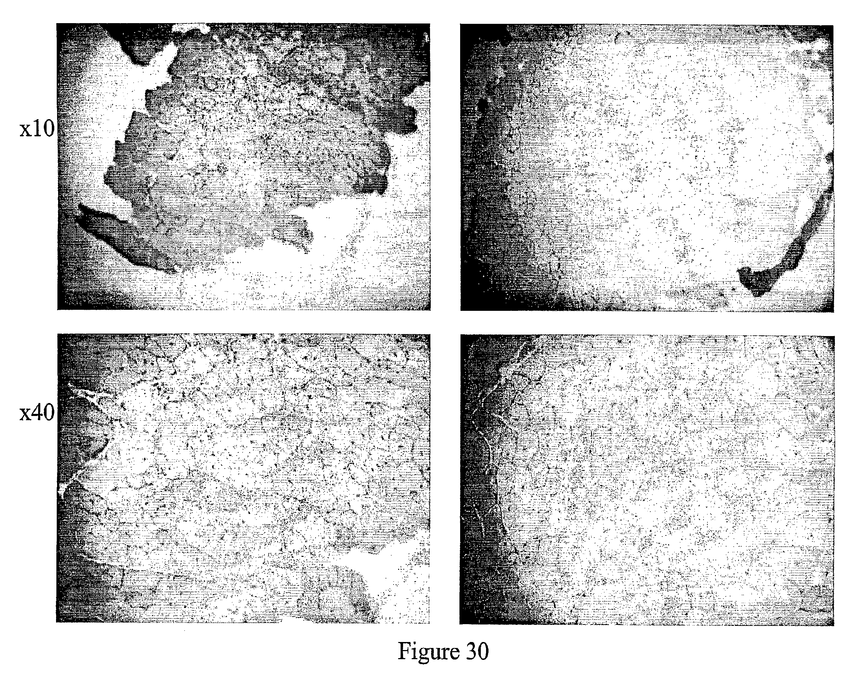

Figure 30 : **Absence de réactions immunitaires cellulaires et humorales 6 mois après transplantation de cellules de la Primo 2CA :** L'existence d'une éventuelle réaction immunitaire **6 mois** après transplantation de cellules Primo 2CA chez une souris mdx immunocompétente a été étudiée en appliquant les mêmes techniques que celles décrites pour la Figure 29.
   Photos de gauche : Tibialis anterior de souris mdx immunocompétente, 6 mois après transplantation de cellules Primo 2CA, marquage à l'aide de l'hématoxyline ;
   Photos de droite : Tibialis anterior de souris mdx immunocompétente, non-traitée (témoin), marquage à l'aide de l'hématoxyline.
   L'absence d'infiltration par les lymphocytes T CD3+ est constatée dans le muscle transplanté par les cellules Primo 2CA, signifiant l'absence de réaction de rejet 6 mois après transplantation.

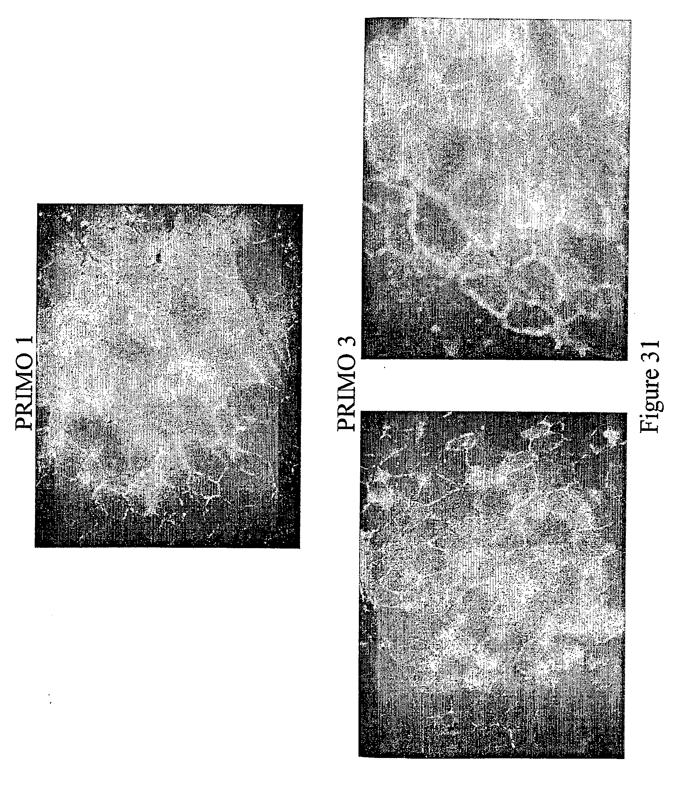

Figure 31 : **Pouvoir de régénération musculaire in vivo des cellules de la primo 1 CA et de la Primo 3 CA après 10 jours de transplantation sans immunosuppresseur chez la souris mdx :** La capacité de régénération musculaire in vivo est évaluée par co-localisation des noyaux humains avec les fibres musculaires re-exprimant la dystrophine. La technique de co-localisation est identique à celle décrite pour les cellules Primo 2CA (voir légendes des Figures 24 à 27), mais les cellules transplantées sont des cellules de la Primo 1CA ou de la Primo 3CA.
   - photo PRIMO1: Tibialis Anterior d'une souris mdx immunocompétente (sans cyclosporine), 10 jours après transplantation de cellules Primo 1CA à 50 et à .120 doublements de population, au nombre de 150 000. L'anticorps utilisé pour la détection de la dystrophine est un anticorps spécifique de la dystrophine humaine (mouse anti-human IgG2a : NCL-DYS3 de Novocastra, dirigé contre l'extrémité N-terminale de la dystrophine humaine)
      photos PRIMO3 : Tibialis Anterior d'une souris mdx immunocompétente (sans cyclosporine), 10 jours après transplantation de cellules Primo 3CA à 45, 80 et 110 doublements de populations, au nombre de 150 000. L'anticorps utilisé pour la détection de la dystrophine est également l'anticorps NCL-DYS3 de Novocastra.

   Après 10 jours de transplantation, un potentiel de régénération musculaire est visible, à la fois pour les cellules de la Primo 1 CA et pour les cellules de la Primo 3 CA.

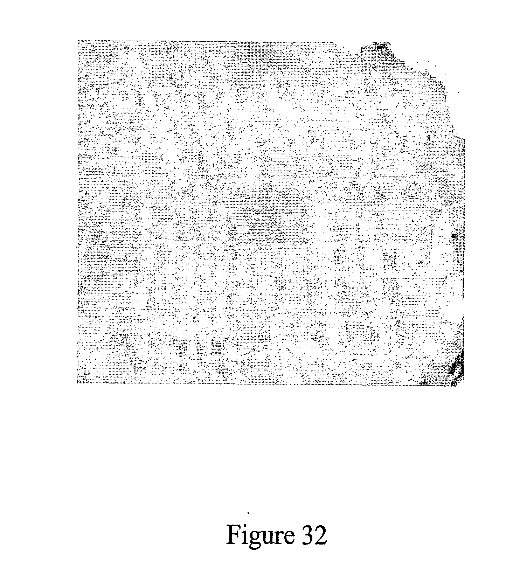

Figure 32 : **Absence de réactions immunitaires cellulaires et humorales 10 jours après transplantation de cellules de la Primo 3 CA :**
   L'existence d'une éventuelle réaction immunitaire 10 jours après transplantation de cellules Primo 3CA chez une souris mdx immunocompétente a été étudiée en appliquant les mêmes techniques que celles décrites pour les cellules de la Primo 2CA (voir légendes des figures Figure 29 et 30).
   photo Primo 3: Tibialis anterior de souris mdx immunocompétente, 10 jours après transplantation de cellules de la Primo 3CA (110 doublements de populations, au nombre de 150 000), marquage à l'aide de l'hématoxyline.
   On observe une absence d'infiltration lymphocytaire avec les cellules de la Primo 3CA, suggérant un comportement identique à celui des cellules de la Primo 2CA.

### EXEMPLES

### 1- Méthode d'obtention et d'expansion in vitro des cellules souches multipotentes du tissu adipeux

### 1.1. Descriptif des prélèvements de tissus adipeux obtenus

Six prélèvements de tissus adipeux de jeunes enfants, âgés de 1 mois à 7 ans, de sexe et de localisation anatomique variables, ont été obtenus.

Le tableau I résume l'origine de chaque prélèvement, le poids de celui-ci ainsi que le nombre de cellules obtenues en utilisant la technique qui sera détaillée plus loin.

**Tableau I : Prélèvements de tissus adipeux humains utilisés pour la production de cellules souches multipotentes**

| **Appellation du prélèvement** | **Sexe** | **Age** | **Localisation anatomique** | **Poids du prélèvement** | **Nombre de cellules obtenues** |
|---|---|---|---|---|---|
| Primo 1 | F | 2 ans 7 mois | Région ombilicale | 300 mg | 400 000 |
| Primo 2 | M | 5 ans | Région pubienne | 400mg | 500 000 |
| Primo 3 | M | 4 mois | Région prépubienne | 210 mg | 400 000 |
| Primo 4 | F | 7 ans | Région inguinale | 2.1 g | 2 000 000 |
| Primo 5 | M | 1 mois | inconnu | 200 mg | 1 000 000 |
| Primo 6 | M | 18 mois | inconnu | 200 mg | 350 000 |

Les Exemples ci-dessous décrivent l'obtention de cellules souches multipotentes à partir des prélèvements Primo 1 à 6. Ces cellules souches ont été obtenues par la mise en oeuvre des étapes suivantes :
▪ isolement de cellules multipotentes à partir du prélèvement
▪ enrichissement *in vitro* de la culture en cellules multipotentes
▪ obtention d'une population de cellules souches quiescentes
▪ induction d'une prolifération intensive de cellules souches

Les cellules souches ainsi obtenues ont été caractérisées par
▪ Mesure de l'activité télomérase
▪ Réalisation de caryotypes à différents passages
▪ Etude de la plasticité cellulaire (différenciation en différents types cellulaires)
▪ Détermination de la présence ou de l'absence de marqueurs cellulaires
▪ Clonage des cellules souches

La méthodologie et les résultats sont décrits en détail ci-dessous.

### 1.2 Méthode d'isolement des cellules multipotentes à partir de tissu adipeux de jeunes enfants

Après l'acte chirurgical, le prélèvement est conservé dans du milieu DMEM (Dulbecco's Modified Eagle's Médium). + 10% de sérum de veau foetal, à température ambiante. Le tissu est rincé dans du PBS (Phosphate Buffer Saline) à 37°C puis égoutté et pesé. Le prélèvement est ensuite émincé très finement pour optimiser l'étape de digestion enzymatique.

Le milieu de digestion est composé de milieu DMEM (Dulbecco's Modified Eagle's Medium) contenant des antibiotiques (100 U/ml de Penicilline et 100µg /ml Streptomycine), 2 mg/ ml de collagénase (Boerhinger référence 103586) et 20mg/ml de sérum albumine bovine fraction 5 (Sigma A référence 2153). Le volume de digestion étant fonction du poids de tissu, on utilise généralement 1ml de milieu de digestion pour 100 à 200 mg de tissu. La digestion s'effectue à 37°C sous agitation lente. Contrairement aux techniques classiquement décrites sur la préparation de préadipocytes humains, la durée de la digestion est très rapide, de 5 à 10 minutes, durée qui correspond à la dissociation complète du tissu par la collagénase. L'activité collagénase est ensuite inhibée par l'addition de sérum de veau foetal (200 µl/ml de milieu de digestion).

On procède alors à la centrifugation de la préparation cellulaire 5 min à 1000 rpm, étape qui va permettre de séparer les adipocytes (qui flottent) des autres types cellulaires contenus dans le tissu adipeux (préadipocytes, cellules souches, cellules endothéliales, péricytes, mastocytes...). Il est important de noter que cette étape de la procédure est effectuée sans filtration, ce qui permet de conserver tous les types cellulaires contenus dans le tissu adipeux (exception faites des adipocytes).

Le culot cellulaire obtenu après centrifugation est resuspendu dans le milieu de culture: DMEM+ 10 % de sérum de veau foetal décomplémenté + antibiotiques (100 U/ml de pénicilline, 100 µg / ml de streptomycine). Le nombre de cellules obtenues est compté. Le rendement cellulaire est variable selon les prélèvements: de 1000 à 5000 cellules par mg de tissu. Les cellules sont ensemencées à haute densité, de 1000 à 3000 cellules par cm2, sur des boites plastiques (polystyrène cristal, Greiner).

Au moment de la mise en culture, deux sous-populations cellulaires sont isolées à partir de leur vitesse d'adhésion. La première sous-population, désignée sous le terme « CA », est constituée de cellules qui adhèrent très rapidement (moins de 12h). La deuxième sous-population, appelée CS, est constituée de cellules qui adhèrent beaucoup plus lentement (de 48 à 72h).

Pratiquement, 12 h après la mise en culture, certaines cellules ont adhéré au plastique. Ces cellules constituent la population CA. Les cellules constituant la population CS, se trouvent au même moment, en suspension dans le milieu de culture. Ce milieu de culture est prélevé et déposé dans une nouvelle boite de culture. Après 72h, les cellules CS ont adhéré.

### 1.3 Enrichissement de la culture en cellules souches multipotentes :

### 1.3.1 Obtention d'une population de cellules souches quiescentes :

Les deux sous-populations CA et CS sont maintenues en culture de la même façon. Ces cellules, à des stades précoces (correspondant environ à 50-60 doublements de population), présentent des caractéristiques similaires en termes de plasticité, prolifération et morphologie.

Les cellules sont entretenues dans le milieu de culture DMEM+ 10% de sérum de veau foetal décomplémenté + antibiotiques (100 U/ml de pénicilline, 100 µg/ml de streptomycine).

Lorsque les cellules arrivent à 80% de confluence, elles sont traitées à la trypsine (Trypsine-EDTA, Invitrogen) et remises en culture dans trois nouvelles boîtes de diamètre identique. La densité d'ensemencement correspondante est de 1000 à 3500 cellules/ cm2. Les cellules ne sont volontairement pas diluées davantage, afin de conserver dans toutes les boites, les cellules multipotentes qui théoriquement se divisent plus lentement que les précurseurs.

Les cellules sont entretenues dans ces conditions jusqu'à ce qu'elles cessent de proliférer. Pour Primo 2, les cellules CA et CS cessent de proliférer à partir du transfert cellulaire 20 (T20), correspondant à 60 doublements de population.

Une coloration Xgal (révélant l'activité β-galactosidase endogène, détectée à pH 6 et traduisant la sénescence des cellules), révèle que la population CS est sénescente alors que la population CA est simplement quiescente (voir Figure 1).

Pour cette étape d'enrichissement, différents milieux de culture ont été testés. Il a notamment été observé que les cellules souches de l'invention ne réagissent pas au « Leukemia Inhibitory Factor » (LIF) (10ng/ml). Le LIF ne produit aucun changement de morphologie ou de prolifération des cellules. Ceci tend à confirmer que les cellules n'expriment pas de récepteur pour LIF (LIF-R⁻)

### 1.3.2. Induction d'une prolifération intensive des cellules souches

Après établissement d'une population de cellules CA quiescentes, l'on rajoute alors au milieu de culture précédemment décrit du bFGF (basic Fibroblast Growth Factor) humain à la concentration de 5 ng/ml de milieu.

Comme l'indique la **Figure 2**, seules les cellules de la population CA répondent de manière efficace au bFGF. En revanche, le bFGF n'a pratiquement pas d'effet sur la population CS à des stades tardifs (c'est à dire après 50 doublements de population).

Ces observations confirment une fois de plus l'état de quiescence de la population CA et l'état de sénescence de la population CS.

Les cellules CA, traitées au bFGF, sont soumises à un traitement à la trypsine à 80% de confluence et diluées cette fois-ci de 5 à 10 fois dans de nouvelles boites de culture identiques.

Deux points supplémentaires sont à préciser sur le bFGF :
- Le bFGF entraîne un changement de la morphologie des cellules. Quiescentes, celles-ci sont applaties et élargies. En présence de bFGF, et donc en phase proliférative, elles prennent une forme fibroblastique (**Figure 3****,** **Figure 12**)
- De plus, l'effet du bFGF est réversible (**Figure 3**).

### 1.4. Congélation des cellules des deux sous-populations CA et CS

Les cellules des deux sous-populations CA et CS sont régulièrement congelées, pour constituer un stock de chaque population cellulaire et pour permettre de suivre son évolution au cours des transferts cellulaires. La cryoconservation ne change pas les propriétés de ces cellules.

De façon pratique, les cellules sont trypsinées, centrifugées et re-suspendues dans le milieu de congélation constitué de sérum de veau foetal additionné de 10% de DMSO. Ces cellules sont ensuite placées à-20°C pendant 1 h, puis à -80°C pendant une nuit et enfin stockées dans l'azote liquide à -180°C.

### 2. Mesure de l'activité télomérase des cellules souches

### 2.1 Méthodologie pour la détermination de l'activité télomérase :

L'activité télomérase est quantifiée par le kit TeloTAGGG Telomerase PCR Elisa ^{PLUS} (Roche).

La quantification de l'activité télomérase se fait en deux étapes :
i) La première étape est une étape d'amplification/élongation (ou TRAP assay) où la télomérase ajoute des motifs télomériques (TTAGGG) à l'extrémité 3' d'un primer biotinylé.
ii) La deuxième étape est la détection et la quantification par Elisa.

Les produits PCR obtenus dans l'étape 1 sont hybridés avec un primer spécifique des extrémités télomériques, marqué à la digoxigenine. De plus les microplaques Elisa sont traitées à la streptavidine, ce qui permet l'immobilisation des produits via la biotine. Les amplicons immobilisés sont détectés avec un anticorps anti-digoxigenine, conjugué à un anti-DIG-HRP et le substrat peroxidase TMB.

L'intensité de la réaction photométrique est estimée en utilisant un lecteur microplaque Elisa (Absorbance à 450 nm avec longueur d'onde de référence 690nm).

On calcule alors l'activité télomérase relative de l'échantillon par rapport à l'activité télomérase d'un contrôle positif (cellules de la lignée HEK293 pour Human Embryonic Kidney 293)

### 2.2 Résultats de la détermination de l'activité télomérase :

En utilisant le kit "*Telo*TAGGG telomerase PCR Elisa Plus" commercialisé par Roche, l'activité télomérase présente dans les 2 sous populations CA et CS de la primo2, a été quantifiée.

Une activité télomérase significative est détectée dans les cellules souches de l'invention. Pour Primo 2CA (T25 : transfert cellulaire 25, correspondant à environ 100 doublements de population), l'activité télomérase est d'environ 20% comparée à l'activité télomérase de la lignée humaine transformée HEK293T (*H*uman *E*mbryonic *K*idney 293 immortalisé par l'Ag T). La lignée HEK293T est utilisée dans ce kit comme référence.

A l'inverse, aucune activité télomérase significative n'est détectée dans les cellules CS, par exemple les cellules de la primo 2 CS (T20) présente une activité d'environ 5% comparée à celle des HEK293T.

### 3. Caryotype des cellules souches

Le caryotype permet l'observation et la classification des chromosomes présents au cours de la métaphase.

### 3.1 Méthodologie pour la détermination du caryotype :

Les métaphases sont obtenues en utilisant les techniques classiques de cytogénétique. Après accumulation des cellules en métaphase par blocage de l'appareil fusorial (incubation en présence de colchicine pendant 3h), on procède à la dispersion chromosomique dans le cytoplasme par l'action d'une solution hypotonique (75 mM KCI pendant 40 min à 37°C) puis à une fixation avec du méthanol/acide acétique (3/1). Les chromosomes sont ensuite identifiés par la technique des bandes R (RHG-banding techniques)

### 3.2 Résultats de la détermination du caryotype:

Le caryotype des cellules a été réalisé. Ces caryotypes ont été effectués sur les 2 sous populations CA et CS, en présence ou non de bFGF et à différents passages (T21, T23 et T34 pour les primo2 CA).

Dans tous les cas, les caryotypes sont tout à fait normaux. Les cellules n'ont donc subi aucun remaniement chromosomique. La **Figure 4** montre le caryotype des cellules de la Primo 2CA.

### 4. Plasticité cellulaire des cellules souches

La plasticité des cellules souches est évaluée selon les techniques suivantes :

### 4.1 Méthodologie pour évaluer la plasticité cellulaire :

### 4.1.1. Conditions de différenciation en différentes types cellulaires :

Les cellules sont trypsinées puis ensemencées à 20 000 cellules / cm2. Les cellules atteignent la confluence 24 à 48h après. La confluence étant une étape critique pour la différenciation, les cellules sont maintenues à confluence 24h supplémentaires avant de procéder à la différenciation (adipocytes, ostéoblastes, myocytes).

### i) Conditions de différenciation en adipocytes

Les cellules confluentes sont incubées dans un milieu DMEM/ Ham's F12 (vol/vol, 1:1) supplémenté de 100U/ml de pénicilline, 100 µg / ml de streptomycine, 5µg/ml d'Insuline humaine (Sigma), 10µg/ml de transferrine humaine (Sigma), 1µM d'activateur de PPAR (par exemple BRL49653), 100 à 250 µM isobutyl-methylxanthine (IBMX) et 1 µM de dexamethasone. Quarante huit à soixante douze heures après, ce milieu est remplacé par le milieu précédemment décrit mais ne contenant plus d'IBMX et de dexamethasone. Ce milieu de différenciation est remplacé tous les 2-3 jours, et ce pendant une période de 15 à 20 jours, correspondant à une différenciation adipocytaire optimale.

### ii) Conditions de différenciation en ostéoblastes

Les cellules confluentes depuis 24h sont incubées avec le milieu de différenciation ostéoblastique composé de DMEM, 100 U/ml de pénicilline, 100 µg / ml de streptomycine, 10% de sérum de veau décomplémenté, 0.1µM de dexaméthasone (SIGMA), 10mM β-glycérophosphate (SIGMA) et de 50µM d'acide ascorbique (SIGMA).

Le milieu est remplacé tous les 2-3 jours et ce pendant une période comprise entre 15 et 20 jours.

### iii) Conditions de différenciation en myocytes

Les cellules confluentes depuis 24h, sont incubées soit dans du milieu DMEM, soit dans le milieu PromoCell, en présence de 2% de sérum de veau décomplémenté et d'antibiotiques (100 U/ml de pénicilline, 100 µg / ml de streptomycine). Le milieu est remplacé tous les 2-3 jours et ce pendant 3 à 6 semaines, particulièrement tous les trois jours, pendant 21 jours.

### iv) Conditions de différenciation en cellules endothéliales

Les cellules sont ensemencées à 20 000 /cm² dans un milieu DMEM contenant 10 ng/ml de VEGF₁₂₁ humain (SIGMA). Le milieu de différenciation est remplacé tous les 2-3 jours, pendant 21 jours.

### 4.1.2 Colorations

### i) Coloration Oil Red O (adipocytes: coloration des lipides intracellulaires)

Après fixation dans une solution PBS/0.25% glutaraldéhyde, les cellules sont incubées pendant 5 minutes à température ambiante dans une solution de Oil Red O 2% (poids/volume). Les cellules sont ensuite lavées puis conservées dans du glycérol 70%.

### ii) Coloration Alizarin Red (ostéoblastes: Calcification de la matrice extracellulaire)

Après fixation dans une solution PBS/025% glutaraldéhyde, les cellules sont incubées 5 minutes à température ambiante avec la solution Alizarin Red 1% (poids/volume). Les cellules sont ensuite lavées à l'eau puis conservées à sec.

### 4.1.3. Analyse transcriptionnelle

### i) Extraction d'ARN

Les ARNs cellulaires sont extraits en utilisant le Tri Reagent (Euromedex, Ref TR-118)

### ii) Northern Blot

20µg d'ARN / puit sont déposés sur un gel d'agarose(1.2%)/MOPS (1X)/ formaldéhyde (1.1M). Après électrophorèse dans du tampon de migration MOPS (1X), les ARN sont transférés sur une membrane de nylon Hybond N+ (Amersham Pharmacia).

La membrane est ensuite hybridée en présence d'une sonde spécifique , marquée au ³²P [dCTP] à l'aide du kit Rediprime TM II Random Prime Labelling system (Amersham Pharmacia).

### iii) RT-PCR

La réaction de Reverse transcription PCR a été réalisée en utilisant le kit OneStep RT-PCR de Qiagen.

### 4.1.4. Analyse de l'expression de marqueurs intracellulaires :

Cette technique a été utilisée pour quantifier le nombre de cellules de la primo2 CA capables de se différencier *in vitro* en myocytes. Le marqueur analysé est la myosine des fibres musculaires rapides (fast twitch myosin ou FT myosin), marqueur tardif de la myogénèse.

Après décollement des cellules, celles-ci sont fixées en présence de PBS/ 1% formaldéhyde pendant 15 min à température ambiante, puis perméabilisées avec une solution de digitonine (10µg/ml de PBS) pendant 7 à 8 min à température ambiante.

On procède ensuite au marquage anticorps, en suivant la protocole décrit pour la détection des marqueurs de surface (voir Exemples 7 et 11 ci-dessous). L'anticorps utilisé est un anticorps de souris conjugué directement à la phycoérythrine et reconnaissant la FT myosine humaine.

### 4.1.5. Immunohistochimie

Les cellules sont fixées avec 4% de paraformaldéhyde pendant 10 min à température ambiante. Lorsque la protéine recherchée est nucléaire (comme par exemple la myogénine), les cellules sont perméabilisées en présence de PBS/0.1% Triton X100 pendant 10 min. On procède ensuite au blocage de l'activité peroxidase endogène, en incubant les cellules avec 3% de H2O2 pendant 5min.

Les cellules sont ensuite incubées avec l'anticorps primaire entre 30 min et 1 h à température ambiante, puis avec l'anticorps secondaire (anti mouse IgG couplé à la peroxidase (Vector Laboratories) ou anti-goat IgG couplé à la peroxidase (Santa Cruz Biotechnology).

Les anticorps primaires utilisés dans nos expériences, von Willebrand factor (vWF) (goat IgG, reconnaissant à la fois le vWF humain, de rat et de souris) (Santa Cruz Biotechnology) et Myogénine (mouse anti human IgG) (Santa Cruz Biotechnology),ont été utilisés au 1 :100.

### 4.2 Résultats de l'analyse de la plasticité cellulaire

### 4.2.1. Plasticité des deux sous populations cellulaires CA et CS à des passages précoces

A des stades précoces (par exemple T1, T5, T7 correspondant à 3, 15 et 21 doublements de population, respectivement), les populations CA et CS présentent les mêmes caractéristiques en terme de plasticité, morphologie et prolifération.

### i) Différenciation en adipocytes

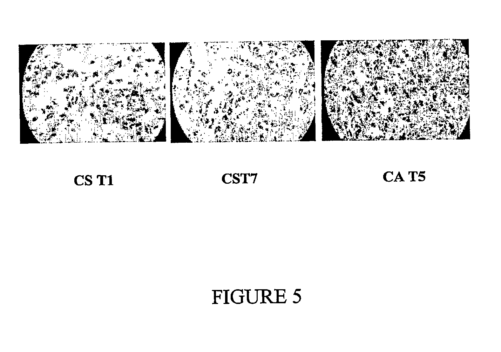

Les résultats pour les expériences impliquant une Coloration Oil Red O sont illustrés dans la **Figures 5** (pour Primo 2CA et Primo 2CS) et la **Figure 14** pour Primo 1CA (40 doublements de populations), Primo 3CA (25 doublements de population) et Primo 6CA (25 doublements de population). En outre, des adipocytes issus de la Primo 1CA, Primo 3CA et Primo 6CA après 50, 40 et 40 doublements de population, respectivement, sont illustrés dans la **Figure 13**. Une comparaison des Figures 13 et 14 démontre clairement que, plus le nombre de doublements de population est élevé, plus la différenciation est homogène.

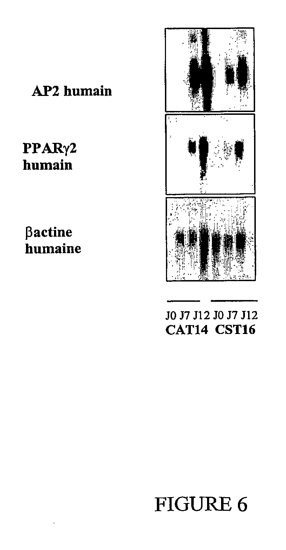

Une analyse par « Northern Blot » **(****Figure 6****)** démontre l'expression transcriptionnelle de marqueurs adipogéniques (aP2 et PPARγ2) au cours de la différenciation : CA T14 (42 doublements de population) et CS T16 (48 doublements de population).

### ii) Différenciation en ostéoblastes

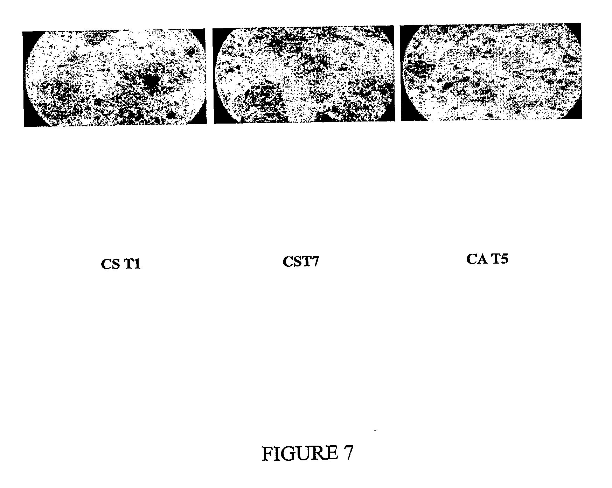

La Figure 7 illustre la différenciation des cellules CS et CA en osteoblastes. Coloration Alizarin Red.

### 4.2.2. Evolution de la plasticité cellulaire à des passages tardifs

Les cellules de la primo 2 CS, à partir du transfert 20 (correspondant à environ 60 doublements de population) entrent en sénescence. Elles perdent simultanément leur potentiel prolifératif et leur capacité de différenciation.

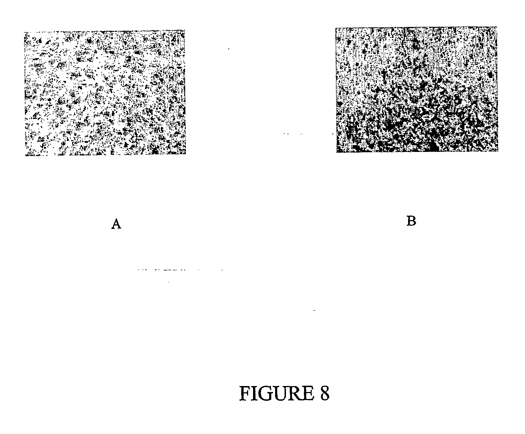

Par contre, les cellules de la population CA, au même stade, deviennent quiescentes. Elles prolifèrent en présence de bFGF et conservent leur plasticité. Cette plasticité reste inchangée à un transfert 40 (correspondant à environ 200 doublements de population). La Figure 8 illustre la différenciation adipocytaire et ostéoblastique pour la Primo2 CA T30 (environ 130 doublements de population).

On conserve également dans la population CA, à des passages tardifs, l'expression transcriptionnelle des différents marqueurs spécifiques soit de la différenciation adipocytaire, soit de la différenciation ostéoblastique. (**Figure 9**) Primo2 CA T32 (140 doublements de population).

### 4.2.3 - Capacité des cellules de la primo2 CA à se différencier en myocytes et cellules endothéliales in vitro

Les cellules de la primo2 CA sont capables, dans des conditions de culture appropriées de se différencier *in vitro,* après 3 semaines environ, en myocytes et en cellules endothéliales.

### i) Différenciation myocytaire

Après 4 jours en présence de milieu de différenciation en myocytes, les cellules de la primo2 CA expriment des marqueurs précoces de la différenciation myocytaire comme la myogénine (marquage en immunohistochimie cf Fig 21). Après 7 jours, l'expression de myogénine n'est plus détectable.

Après 21 jours, 95 % des cellules de la primo2 cultivées dans ce milieu, expriment un marqueur tardif de la différenciation myocytaire, à savoir la myosine des fibres musculaires rapides ou Fast Twitch myosin (marquage intracellulaire et analyse au FACs, cf fig 22).

### ii) Différenciation en cellules endothéliales

Contrairement aux cellules adipocytaires, ostéoblastiques et myocytaires qui appartiennent au "Limb bud mesoderm", les cellules endothéliales dérivent du mésoderme visceral.

Après 21 jours de maintien dans un milieu composé de DMEM et de hVEGF121 (10ng/ml), les cellules de la primo2 CA expriment le von Willebrand factor, marqueur spécifique des cellules endothéliales (immunohistochimie, cf fig23).

### 5. Caractérisation de la fonctionnalité adipocytaire par dosage enzymatique

### 5.1 Méthodologie pour caractériser la fonctionnalité adipocytaire

### 5.1.1 Mesure de l'activité Glycérophosphate Déshydrogénase (GPDH)

On procède dans un premier temps à la lyse des cellules dont on veut mesurer l'activité GPDH. Le principe du dosage est résumé sur ce schéma:

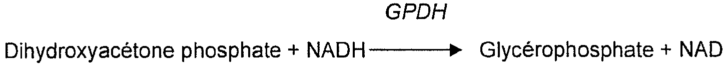

On détermine la vitesse initiale de disparition du NADH à 340nm (en présence de NADH, DHAP et du lysat cellulaire), ce qui permet de calculer la quantité de substrat dégradé, d'où l'activité enzymatique spécifique (après dosage des protéines).

La lecture se fait sur un spectrophotomètre permettant de faire des cinétiques (KONTRON Uvicon 860 thermostaté à 37°C).

### 5.9.2. Test de lipolyse

Ce test consiste à mesurer le glycérol radiomarqué libéré par les adipocytes en présence d'agonistes de récepteurs □adrénergiques. La méthode utilisée est celle décrite par Bradley DC et Kaslow HR (Anal Biochem, 1989, 180,11-16). Le glycérol libéré est phosphorylé en présence de glycérokinase, et d'ATP et d'ATP marqué au P³² sur la position γ. L'ATP résiduel est ensuite hydrolysé en milieu acide à 90°C et précipité avec du molybdate d'ammonium et de la triéthylamine. La radioactivité incorporée sous forme de glycérophosphate marqué au P³² est estimée par comptage au compteur β et les valeurs ont exprimées en pmol grâce une courbe étalon.

### 5.2 Résultats de la caractérisation de la fonctionnalité adipocytaire

### 5.2.1. Activité Glycérophosphate Déshydrogénase (GPDH)

**Cellules primo2 CA** (T24 en présence de bFGF humain) :
*Après 11 jours de différenciation* (2 expériences)
Contrôle: 77 nmol/min/mg de protéine
En présence d'un agoniste de PPARγ (BRL49653): 290nmol/min/mg de protéine

*Après 16 jours de différenciation* (3 expériences)
Contrôle: 20 nmol/min/mg de protéine
En présence d'un agoniste de PPARγ (BRL49653): 390 nmol/min/mg de protéine

**Cellules primo2 CS** (T22 en présence de bFGF humain) :
*Après 13 jours de différenciation* (3 expériences)
Contrôle: 22 nmol/min/mg de protéine
En présence d'un agoniste de PPARγ (BRL49653): 30 nmol/min/mg de protéine

### 5.2.2. Capacité de lipolyse des cellules de la primo 2 CA

Les lipolyses ont été réalisées, sur des cellules primo2 CA T32 avec des agonistes spécifiques des différents récepteurs β adrénergiques, à savoir:
Isoprotérénol: β1, β2 adrénergique
Dobutamine: β1 adrénergique
Terbutaline: β2 adrénergique
CL316243 β3 adrénergique:

Les vitesses de lipolyse obtenues sont les suivantes (d'après la courbe de glycérol étalon) :
Contrôle: 5.76 nmol/h/mg de protéine
Dobutamine: 60.1 nmol/h/mg de protéine
Terbutaline:93.78 nmol/h/mg: 60.1 nmol/h/mg de protéine
CL316243: 17.1 nmol/h/mg de protéine

Les résultats sont illustrés dans la **Figure 10**.

Les expériences de lipolyse montrent la présence de récepteurs β1 et β2 adrénergiques, et l'absence de récepteurs β3 adrénergiques, résultats en accord avec les observations *in vivo* (Galitzky et al; (1997) British J. Pharmacol 122: 1244-1250).

### 6. Caractérisation de la fonctionnalité des ostéoblastes par détection du calcium associé à la matrice extracellulaire

### 6.1 Méthodologie pour la caractérisation de la fonctionnalité des ostéoblastes :

Pour détecter le calcium sécrété par les ostéoblastes, les cellules sont cultivées dans le milieu de différenciation ostéoblastique précédemment décrit.

Après différenciation optimale, le tapis cellulaire est lysé avec une solution 0.1 N NaOH pendant 45 min. On procède ensuite à une étape de neutralisation, en rajoutant du HCL 1 N (0.2 vol/ 1 vol NaOH). Les boites, où restent toujours la matrice extracellulaire, sont séchées puis incubées avec la solution du "SIGMA calcium détection kit". La quantité de calcium sécrété par les ostéoblastes est quantifiée par lecture de cette solution au spectrophotomètre (DO575)

### 6.2. Résultats : Fonctionnalité des ostéoblastes

La fonctionnalité des ostéoblastes a été mise en évidence par la technique de détection du calcium associé à la matrice extracellulaire (**Figure 11**)**.**

Nous pouvons également souligner l'importance du lot de sérum de veau dans la différenciation ostéoblastique. Ceci reflète le rôle crucial dans la différenciation ostéoblastique de certaines cytokines, hormones ou facteurs de croissance non caractérisés et présents dans des proportions variables selon les lots de sérum.

### 7. Marquage cellulaire et analyse en cytométrie de Flux

Le caractère HLA Classe I négatif des cellules souches de l'invention a été mis en évidence par analyse en cytométrie de flux, utilisant un système de simple marquage classique :

### 7.1 Simple marquage

Les cellules sont décollées puis lavées dans du PBS. Après centrifugation, les cellules sont re-suspendues et incubées avec l'anticorps primaire à la concentration de 10 µg/ml pendant 30 min à 4°C. Les anticorps utilisés sont soit un anticorps monoclonal de souris, dirigé contre les molécules HLA de classe I (W6/32, Novocastra), soit un anticorps IgG de souris (Santa Cruz) utilisé comme contrôle négatif. Le nombre de cellules utilisées par condition est de 5 x 10⁵ à 10⁶. Les cellules employées pour cette analyse sont les suivantes :

| | |
|---|---|
| HeLa : | Cellules humaines tumorales (HLA Classe I positif : contrôle positif). |
| SVF: | Tissu adipeux adulte, pas de doublement de population (HLA Classe I positif) |
| Primo 2CA : | 120 doublements de population |
| Primo 2CS : | 45 doublements de population |

Les cellules sont ensuite lavées puis incubées pendant 20 min à 4°C avec un anticorps (dit secondaire) qui est un anti IgG de souris couplé au FITC (0.2µg/10⁶ cellules) (Caltag).

Les cellules sont ensuite lavées et leur fluorescence analysée par cytométrie en flux (Scan FACS Becton Dickinson).

Les résultats sont illustrés dans la Figure 15, et montrent que les cellules souches de l'invention (par exemple Primo 2CA) présentent un niveau d'expression de molécules HLA de Classe I non-détectable par cytométrie de flux en simple marquage. Les cellules souches de l'invention sont donc « HLA Classe I négatif ».

Cette expérience de cytométrie de flux a également été mise en oeuvre avec les cellules souches de la Primo1CA et de la Primo3CA, ainsi qu'avec les cellules de la Primo 2CS (Figure 15). Dans tous les cas, le niveau d'expression de surface de HLA Classe I est négatif.

### 8. Obtention de clones multipotents à partir des cellules CA

Les cellules de la Primo 2 CA ont été ensemencées en condition de dilution limite, à savoir 1/3 de cellules par puits de plaques de 24 puits, puis maintenues en présence de 10% FCS contenant 5ng/ml bFGF.

Dix jours après, des clones ont été isolés et amplifiés. Ces clones conservent leur phénotype non-différencié jusqu'à la mise en condition de différenciation. Leur capacité de différenciation en adipocytes et ostéoblastes est alors mise en évidence.

La Figure 16 montre deux clones, CA1 et CA3, qui ont été mis dans le milieu de culture permettant la différenciation en adipocytes et en ostéoblastes. Les adipocytes sont révélés par le colorant à l'huile rouge et les ostéoblastes par le rouge Alizarin. Les clones analysés par cytométrie de flux se révèlent également HLA Classe I et II négatifs

### 9. Expression d'un transgène dans les cellules souches

Les cellules souches de l'invention, notamment les cellules du clone CA1 (obtenu selon l'Exemple 7) ont été transduites au stade 21 doublements de population, par un rétrovirus exprimant un gène de résistance à un antibiotique, la puromycine, et le gène rapporteur LacZ sous le contrôle d'un promoteur LTR.

Les virions infectieux sont produits à partir de cellules 293 transfectées de façon stable avec un vecteur PVPack-GP (contenant les séquences gag et pol), que l'on co-transfecte avec le plasmide pFB-Neo-lacZ et le vecteur PVPack-VSVG expressing vector, contenant la protéine G du virus de la stomatite vésiculaire.

**La** **figure 17** montre que les cellules transduites puis sélectionnées en présence de puromycine expriment toutes le gène LacZ révélé in situ par l'activité β-galactosidase.

### 10. Expression de Oct-4, d' ABCG2 et de Rex-1 dans les cellules souches CA

Oct-4 est un facteur de transcription exprimé spécifiquement dans les cellules souches embryonnaires de souris et est indispensable au maintien de leur pluripotentialité. Il a été montré également que Oct-4 est exprimé par les cellules souches embryonnaires humaines.

L'expression transcriptionnelle de Oct-4 a été mise en évidence dans les cellules souches de l'invention. Les ARN sont extraits à partir des cellules CA (populations homogènes et populations clonales) et l'expression de Oct-4 est amplifiée par RT-PCR, puis détectée par hybridation. Les conditions de PCR sont: 94°C, 1 mn ; 57°C, 1 mn ; 72°C, 1 mn pendant 45 cycles. -RT : contrôle négatif.

De même, l'expression transcriptionnelle d'ABCG2 a été mise en évidence dans des cellules CA (populations homogènes et populations clonales). Les ARN sont extraits à partir des cellules CA et CA1 et l'expression de ABCG2 est amplifiée par RT-PCR, puis détectée par hybridation. Les conditions de PCR pour ABCG2 sont: 94°C, 1 mn; 60°C, 1mn; 72°C, 1mn pendant 31 cycles.

**La** **Figure 18** montre les résultats obtenus avec les cellules Primo 2CA du transfert 16 (48 doublements de population) et les cellules du clone Primo 2CA1 du transfert 5 (15 doublement de population). Elles expriment Oct-4 et ABCG2.

Les cellules primo 2 CA expriment également le facteur de transcription Rex-1 (figure 18, partie droite). Le facteur de transcription Rex-1 est un marqueur spécifique de cellules souches embryonnaire de souris et humaines. Les conditions de PCR pour Rex-1 sont les suivantes : 94°C 1 min., 60°C 1 min., 72°C 1 min., nombre de cycles 31, 72° 5min 1 cycle.

### 11. Caractérisation des cellules CA de l'invention en termes de marqueurs de surface

La caractérisation des cellules CA de l'invention, particulièrement les cellules de la Primo 2CA, a été approfondie en termes de marqueurs de surface, à l'aide de la technique de la cytométrie en flux.

### 11.1. Méthodologie pour l'analyse de l'expression des marqueurs de surface par cytométrie de flux:

Le protocole de marquage a été décrit précédemment (voir Exemple 7). Les anticorps utilisés dans ce contexte sont les suivants :
HLA de classe I conjugué à la fluorescéine (FITC) ;
HLA-DR (HLA classe II) conjugué à la phycoérythrine (PE) ;
CD-3 (marqueur des lymphocytes T) conjugué à PE ;
CD13 (marqueur des cellules stromales de la moelle osseuse, cellules endothéliales, progéniteurs précoces des granulocytes / monocytes et de leur descendance) conjugué au FITC

### 11.2 - Résultats de la caractérisation des cellules CA de l'invention en termes de marqueurs de surface :

Les cellules de la primo 2 CA sont négatives en surface pour l'expression de CD3, d'HLA classe I et d'HLA-DR. Elles sont par contre CD13 positives (marqueur exprimé entre autres dans les cellules stromales de la moelle osseuse (cf Fig 20)

L'absence en surface des molécules HLA de classe I et II suggèrent fortement la non immunogénicité des cellules de la primo2 CA.

Il est intéressant de remarquer que les cellules de la primo 2 CA diffèrent des cellules humaines de la moelle osseuse décrite par Reyes et al (Blood, Nov 2001).

Ces cellules appelées MPC pour "Mesodermal Progenitor Cells" sont HLA classe I et classe II négatives et CD13 positives uniquement lorsqu'elles sont cultivées à faible densité, dans un milieu de culture contenant de faible concentration de sérum de veau foetal (2%) avec présence obligatoire d'EGF et de PDGF.

Par contre, cultivées en présence de 10% de sérum de veau foetal (concentration utilisée pour amplifier les cellules de l'invention, en particulier les cellules de la primo2 CA), les hMPCs présentent le phénotype inverse, à savoir HLA classe I et HLA-DR positives et CD13 négatives. Les hMPCs cultivées en présence de 10% de sérum de veau ou de bFGF (FGF-2) perdent leur potentiel de prolifération très rapidement et meurent.

### 12. Différenciation des cellules souches CA de l'invention en cellules endothéliales et en myocytes in vivo :

Il a été démontré dans l'exemple 4 que les cellules de l'invention sont capables de se différencier *in vitro,* après trois semaines en cellules endothéliales et en myocytes.

Les exemples présentés ci-dessous démontrent que les cellules humaines CA de l'invention (particulièrement les cellules de la Primo 2CA, de la Primo 1CA et de la Primo 3CA) injectées dans le muscle de souris mdx, modèle animal des myopathies de Duchenne chez l'homme, sont capables de régénérer des fibres normales après 10 jours seulement. De façon surprenante, ces cellules ne sont pas rejetées lorsqu'elles sont transplantées chez ces souris non immuno-supprimées. De telles cellules, par leur capacité de régénération *in vivo* et leur absence d'immunogénicité, offrent de nombreuses perspectives thérapeutiques dans un contexte allogénique.

### 12.1 Protocole de transplantation et analyse de la régénération in vivo par immunofluorescence et FISH (Fluorescence In Situ Hybridization)

### 12.1.1. Protocole de transplantation

Pour analyser le potentiel de régénération in vivo des cellules CA de l'invention (plus particulièrement des cellules la primo2 CA, de la Primo 1CA et de la Primo 3CA), le modèle animal, des souris mdx (C57BL/10ScSn DMD^{mdx}/J), a été utilisé.

Ces souris mdx (X-chromosome -linked muscular dystrophy) constitue un bon modèle animal pour étudier les myopathies de Duchenne chez l'homme, car elle présente une mutation ponctuelle du gène de la dystrophine (localisé sur le chromosome X), entraînant la non- traduction de la dystrophine. Chez l'homme, l'absence de dystrophine, protéine au rôle mal connu, entraîne une cascade d'événements peu compris à ce jour, provoquant la disparition progressive des fibres musculaires et le décès.

Pour les expériences *in vivo* de régénération musculaire, les cellules suivantes ont été utilisées :
▪ des cellules de la primo 2 CA obtenues entre 80 et 160 doublements de population (plus précisément 80, 120 et 160 doublements de population) ;
▪ des cellules de la Primo 1CA obtenues à 50 et à 120 doublements de population ;
▪ des cellules de la Primo 3CA à 45, à 80 et 110 doublements de populations.

Dans toutes les expériences, des souris mdx âgées de 3 à 4 mois, de sexe indifféremment masculin ou féminin, ont été utilisées.

Le muscle du tibia antérieur gauche est transplanté avec 150 000 cellules de la primo 2 CA, de la Primo 1CA ou de la Primo 3CA, repris dans un volume de 50µl de HBSS (Hank's Buffered Saline solution) Le même volume de HBSS est injecté dans le muscle droit servant de contrôle négatif.

Pour analyser le potentiel de régénération musculaire des cellules de l'invention, nous avons dans un premier temps, traité les souris transplantées avec un agent immunosuppresseur, la cyclosporine, pour éviter le risque de rejets immunitaires. L'administration de cyclosporine se fait par voie intrapéritonéale, une fois par jour à la concentration de 10mg/kg (poids de l'animal)/jour et ce à partir de la transplantation.

Les souris mdx transplantées et traitées simultanément à la cyclosporine sont sacrifiées après 10 jours.

En parallèle, pour tester la non immunogénicité des cellules de la primo2 CA, de la Primo 1CA ou de la Primo 3CA, les inventeurs ont utilisé le même protocole de transplantation mais sur des souris mdx ayant un système immunitaire normal, c'est à dire non traitées à la cyclosporine.

Les muscles transplantés, ainsi que les muscles contrôles (injectés uniquement avec 50µl HBSS), sont récupérés et congelés dans l'isopentane puis l'azote liquide.

### 12.1.2 Détection de la dystrophine par immunofluorescence

Des coupes sériées de 12 µm sont préparées à partir des muscles congelés et déshydratées par passages successifs de 10minutes dans l'éthanol 50, 75 et 100%.

Pour réaliser le marquage à la dystrophine, les coupes congelées sont fixées au méthanol/ acide acétique glacial (70/30, v/v) pendant 15 min à température ambiante. Après lavage au PBS (Phosphate- buffered Saline) et incubation pendant 30 min dans une solution de blocage (PBS+3% BSA (Bovine Serum Albumine)), les coupes sont incubées pendant une heure avec un anticorps spécifique de la dystrophine humaine (mouse anti-human IgG2a, Novocastra NCL-DYS3), ou un anticorps qui reconnait à la fois la dystrophine humaine, de rat et de souris (mouse IgG1 NCL-DYS2 Novacastra). Pour réduire le bruit de fond, l'anticorps est préalablement couplé à la fluorescéine (Alexa Fluor 488), en utilisant le "Zenon Alexa Fluor 488 Mouse IgG2a ou IgG1 Labeling Kit ", selon l'anticorps (Molecular Probes).

Les coupes sont ensuite lavées au PBS puis à l'eau et analysées par microscopie à fluorescence (Olympus BH2).

### 12.1.3. Détection des noyaux humains transplantés par FISH (Fluorescence In Situ Hybridization)

Les lames sont déshydratées par passages successifs de 2 min, dans des bains d'éthanol à concentrations croissantes (70, 80 et 100%), puis dénaturées à 73°C dans une solution 70% formamide/2XSSC (solution saline citratée) pendant 2min 30s. Les lames sont ensuite réhydratées (bains d'éthanol de concentrations décroissantes 100, 80 et70%) avant de procéder à l'hybridation.

La sonde utilisée pour détecter les noyaux humains est une sonde spécifique de tous les centromères humains (α-Satellite) couplée à la digoxigénine (CP5095-DG.5, Appligene Oncor).

La sonde, préalablement diluée dans le tampon d'hybridation Hybrisol VI (Appligène Oncor) (1 µl de sonde pour 10 µl de tampon), est dénaturée pendant 5 min à 72°C puis déposée sur les lames. L'étape d'hybridation se fait à 37°C , dans une chambre humide pendant 12h.

Les lames sont ensuite lavées : 1 lavage en 50% formamide/2XSSC à 43°C pendant 15 min suivi d'un lavage en 2XSSC à 37°C pendant 8 minutes.

L'étape ultérieure est une étape de détection, consistant à l'application sur les lames d'un anticorps anti-digoxigénine couplé à la rhodamine (Appligène Oncor) (5 min à l'obscurité). Avant l'analyse des coupes, on procède à la coloration de l'intégralité des noyaux en utilisant une solution de DAPI (coloration bleu).

Les lames sont ensuite observées au microscope à fluorescence (Axiophot Zeiss) avec une lampe 100 watts et un système de filtres (Perceptive Scientific International).

### 12.1.4. Double marquage Immunofluorescence /FISH

Pour pouvoir co-localiser les noyaux humains avec les fibres musculaires réexprimant la dystrophine, les inventeurs ont procédé à un double marquage (dystrophine/noyaux humains). Pour ce faire, l'étape de détection de la dystrophine a été effectué avant de procéder au marquage des noyaux humains par FISH.

### 12.2- Résultats : Capacité de régénération musculaire et non immunogénicité des cellules CA de l'invention in vivo :

### 12.2.1 Capacité de régénération musculaire in vivo

De nombreuses études concernant la multipotentialité des cellules souches adultes *in vivo* ont été remises en question, notamment après la mise en évidence du pouvoir de fusion de ces cellules (Terada et al, Nature 2002 ; Wurmser et al, Nature, 2002 et Ying et al, Nature, 2002). De plus, certains travaux suggèrent que la capacité de ces cellules à exprimer des marqueurs spécifiques du tissu où elles ont été transplantées, est un évènement extrêmement rare (Wagers et al, Science, 2002 ; Morshead et al, Nature, 2002).

Pour éviter les problèmes évoqués ci dessus, des souris mdx, modèle animal des myopathies de Duchenne, ont été utilisées. Ces souris sont déficientes en dystrophine (mutation ponctuelle au niveau du gène) (En réalité, il existe un faible nombre de fibres révertantes réexprimant de la dystrophine mais le pourcentage de ces fibres ne dépasse pas 1 % (Hoffman et al., J Neurol Sci, 1990 ; Gillis, J Muscle Research and Cell Motility, 1999)).

Dans un premier temps, pour éviter tout phénomène de rejet, les souris mdx ont été traitées avec un agent immunosuppresseur, la cyclosporine.

L'injection d'un faible nombre de cellules de la primo2 CA (100 000 à 150000 cellules) dans le muscle Tibialis Anterior se traduit par une restauration de la dystrophine dans environ 50% des fibres, en seulement 10 jours. Ces fibres positives sont regroupées sous forme de clusters (correspondant sans doute au point d'injection).

En accord avec les résultats précédents, en utilisant la technique de FISH, les noyaux humains avec les fibres musculaires positives pour la dystrophine (cf Fig 24 et 25), ont pu être localisés.

Des résultats similaires sont obtenues pour des cellules de la primo 2 CA entre 60 et 160 doublements de populations, traitées préalablement ou non au bFGF (FGF-2). Des résultats similaires ont été obtenus avec le clone CA1, dérivé de la population primo2 CA.

### 12.2.2 Non immunogénicité des cellules CA in vivo :

Contrairement à la plupart des cellules somatiques, les cellules CA de l'invention sont dépourvues de marqueurs HLA classe-I (voir Exemple 7) et de marqueurs HLA Classe-II en surface (Exemple 11). Ce phénotype extrêmement rare suggère fortement la non-immunogénicité des cellules CA de l'invention.

Pour valider la non immunogénicité de ces cellules in vivo, une approche expérimentale similaire à celle décrite en section 12.2.1 mais en utilisant des souris mdx non immunosupprimées, a été utilisée.

Après 10 jours de transplantation, le muscle des souris transplantées et non immunosupprimées réexpriment des taux de dystrophine comparables à celui des souris traitées à la cyclosporine. Un potentiel de régénération musculaire est constaté, à la fois pour les cellules de la Primo 2CA, et pour les cellules de la Primo 1 CA et de la Primo3CA, en absence d'immunosuppresseur (cf Figure 24 pour les cellules de la Primo 2CA et Figure 31 pour les cellules de la Primo 1 CA et de la Primo 3CA).

Après 50 jours de transplantation, en absence d'immunosuppresseur, le nombre de fibres musculaires positives pour la dystrophine continue à augmenter dans le tibialis anterior injecté mais des fibres se retrouvent également dans d'autres muscles comme le gastrocnémius (cf Figure 25). Ces résultats suggèrent fortement que les cellules de la primo2 CA sont capables non seulement de régénérer le muscle au point d'injection mais également de migrer pour réparer les muscles environnants. Une augmentation du pourcentage de noyaux périphériques d'origine humaine à l'intérieur des fibres, et une diminution du pourcentage de noyaux centraux a été observée entre 10 et 50 jours après transplantation (de 73% à 85%, et de 27% à 15% respectivement), indiquant que les cellules injectées participent à la différenciation terminale des myocytes.

A l'aide de la technique de FISH, les noyaux humains sont toujours présents et colocalisés avec les fibres dystrophine-positives. De plus, la localisation d'un certain nombre de noyaux humains à la périphérie externe des fibres positives suggèrent la présence de cellules satellites humaines et / ou de cellules endothéliales d'origine humaine.

En comparaison, la transplantation en grande quantité de myoblastes humains (4 millions) dans des souris mdx non immunosupprimés se traduit par un rejet total après un mois (Huard et al, Muscle and Nerve, 1994) et donc absence de régénération musculaire.

Après 80 jours de transplantation, le nombre de fibres musculaires positives pour la dystrophine continue à augmenter dans le muscle injecté, et des fibres se retrouvent toujours dans le gastrocnémius (cf Figure 26).

Après 6 mois de transplantation, plus de 80% des fibres expriment de la dystrophine humaine, contre 50% à des temps de transplantation plus précoces (Figure 27). En outre, l'on constate également au sein de la fibre, une expression beaucoup plus régulière de la dystrophine, en comparaison à des temps plus précoces de transplantation où l'expression de la dystrophine est irrégulière au sein de la même fibre. De plus le muscle transplanté présente une amélioration nette au niveau de la morphologie des fibres (morphologie des fibres plus régulière et absence de nécrose, processus important dans les souris mdx de cet âge).

Ces observations indiquent que les cellules transplantées ne donnent lieu à aucune réaction de rejet chez la souris immunocompétente. Le caractère non-immunogène des cellules transplantées a en effet été démontré à l'aide d'une coloration à l'hématoxyline. Aucune réaction de rejet (absence d'infiltration par les lymphocytes T CD3+) n'est observée après 10 jours, (Figure 29), 50 jours, 80 jours (résultats non illustrés), ou après 6 mois de transplantation de cellules de la Primo 2CA (voir Figure 30). De même, l'absence d'infiltration lymphocytaire peut également être constatée 10 jours après transplantation des cellules de la primo 1CA et de la Primo 3CA, confirmant l'absence de réaction de rejet. La Figure 32 illustre les résultats obtenus avec les cellules de la Primo 3CA. Les mêmes résultats ont été obtenus avec les cellules de la Primo 1CA. Le comportement immunoprivilégié des cellules de la primo 1CA et de la Primo 3CA est donc identique à celui observé chez la Primo 2CA. Ces résultats ont également été confirmés en utilisant un anticorps anti CD-3, démontrant l'absence d'infiltration lymphocytaire.

En revanche, la transplantation de cellules stromales-vasculaires humaines non purifiées, isolées à partir de tissu adipeux humain induit une réaction immunitaire cytotoxique et humorale (Fig. 29(c) et (c')).

En conclusion, après six mois de transplantation, les cellules entraînent une nette amélioration du muscle transplanté avec un fort pourcentage de fibres exprimant la dystrophine humaine et une absence du processus de nécrose que l'on observe dans les souris mdx non-traitées du même age, et cela en absence d'immunosuppresseur.

L'origine humaine de la dystrophine exprimée dans les myofibres du muscle transplanté a été démontrée, par immunodétection comparative, utilisant d'une part un anticorps spécifique pour la dystrophine humaine (dirigé contre l'extrémité N-terminale de la dystrophine humaine : mouse anti-human IgG2a : NCL-DYS3 de Novocastra), et d'autre part un anticorps capable de reconnaître à la fois la dystrophine humaine et la dystrophine murine (dirigé contre l'extrémité C-terminale de la dystrophine humaine et murine : mouse anti-human IgG1 : NCL-DYS2 de Novocastra).

Les résultats de cette immunodétection comparative sont illustrés dans la Figure 28. La présence de myofibres exprimant de la dystrophine, et la localisation subcellulaire dans le tibialis anterior 10 jours après transplantation est visible. La similarité entre les Figures 28(a) et (b) indique l'origine humaine de la dystrophine exprimée. La dystrophine humaine est localisée sous le sarcolemne. En revanche, la collagène III de souris est présente dans l'espace extracellulaire entre les myofibres (figures 28(c) à (e)).

Les mécanismes impliqués dans la tolérance des cellules CA de l'invention, dans un contexte xénogénique, c'est à dire dans un organisme immunologiquement très différent (souris mdx), restent encore à élucider.

On peut toutefois supposer qu'un certain nombre de cellules situées à la périphérie externe des fibres musculaires joue un rôle clé dans cette tolérance. Ces cellules peuvent jouer un rôle d'immunosuppression locale en synthétisant des facteurs immunosuppressifs comme par exemple des cytokines anti-inflammatoires type Th2 comme IL10 et/ou en exprimant à leur surface des protéines conduisant à l'absence de reconnaissance par les lymphocytes alloréactifs de l'hôte (Jorgensen et al, Engineering mesenchymal stem cells for immunotherapy, Gene Therapy 10, 928-931 (2003)).

Ces cellules peuvent également induire une tolérance généralisée en permettant une re-éducation du système immunitaire de l'hôte. La présence de cellules souches CA humaines dans le thymus et la rate de l'hôte renforce ce type d'hypothèse (Fändrich F et al, « Preimplantation-stage stem cells induce long-term allogeneic graft acceptance without supplementary host conditioning », Nat. Med 8, 171-178 (2002).

Ces résultats démontrent l'immunoprivilège des cellules humaines CA de l'invention qui sont capables de régénérer le muscle sans être rejetées. Ces cellules offrent ainsi de nombreuses perspectives de thérapies cellulaires en allotransplantation. Notamment, pour les maladies génétiques comme les myopathies où les autotransplantations en l'état sont impossibles, l'utilisation de cellules similaires à la primo2CA constituerait une bonne alternative thérapeutique.

## Revendications

1. Cellule souche humaine multipotente et adulte isolée, **caractérisée en ce qu'**elle présente :
i) une activité télomérase endogène d'au moins 20% de l'activité télomérase de la lignée humaine transformée HEK293T,
ii) un phénotype HLA Classe I négatif, y compris en présence de 10% de sérum de veau foetal,
iii) un caryotype normal,
iv) une capacité à rentrer en quiescence après 50 à 80 doublements de populations,
v) une capacité d'autorenouvellement conservée pendant au moins 130 doublements de population, et
vi) un taux de sénescence inférieur à 0.05% au stade de 60 doublements de population.

2. Cellule souche selon la revendication 1, **caractérisée en ce qu'**elle a une capacité d'autorenouvellement conservée pendant au moins 200 doublements de population.

3. Cellule souche selon la revendication 1 ou 2, **caractérisée en ce qu'**elle est susceptible d'être isolée à partir de tissu adipeux humain.

4. Cellule souche selon l'une quelconque des revendications précédentes, **caractérisée en ce qu'**elle est capable de se différencier en cellule d'origine endodermique ou ectodermique ou mésodermique.

5. Cellule souche selon la revendication 4, **caractérisée en ce qu'**elle est capable de se différencier en adipocyte, en ostéoblaste, en myocyte, en chondrocyte ou en cellule endothéliale.

6. Cellule souche selon l'une quelconque des revendications précédentes, **caractérisée en ce qu'**elle exprime le facteur de transcription Oct-4, et/ou Rex-1.

7. Cellule souche selon l'une quelconque des revendications précédentes, **caractérisée en ce qu'**elle peut exprimer au moins un transgène.

8. Cellule souche selon l'une quelconque des revendications 1 à 7 **caractérisée en ce qu'**elle présente le phénotype suivant :
HLA classe I négatif
HLA classe II négatif
CD3 négatif
CD13 positif

9. Cellule souche selon l'une quelconque des revendications 1 à 8 **caractérisée en ce qu'**elle présente un phénotype CD13 positif en présence de 10% de sérum de veau foetal.

10. Cellule selon la revendication 8" **caractérisée en ce qu'**après avoir atteint la quiescence, elle présente, de façon stable, le phénotype suivant *in vitro* :
HLA classe 1 négatif,
HLA classe II négatif,
CD3 négatif,
CD13 positif,
LIF-R négatif,
Oct-4 positif,
Rex-1 positif, et
ABCG2 positif.

11. Cellule selon la revendication 10, **caractérisée en ce qu'**elle présente *in vivo* une capacité à migrer à l'état non différencié, et ne donne pas lieu à une réaction de rejet plus de 10 jours après transplantation dans un mammifère immunocompétent.

12. Procédé d'obtention de cellules souches humaines multipotentes, comprenant les étapes suivantes :
- mise en culture de cellules provenant d'un prélèvement de tissu adipeux humain,
- sélection de deux sous-populations cellulaires, dites population « CA » et population « CS », la population « CA » présentant une vitesse d'adhésion inférieure à 12 heures, et la population « CS » présentant une vitesse d'adhésion supérieure à 12 heures,
- enrichissement de la population « CA » jusqu'à l'obtention d'une population de cellules quiescentes,
- induction d'une prolifération des cellules souches de la population « CA ».

13. Procédé selon la revendication 12, comprenant les étapes suivantes :
a) digestion enzymatique d'un prélèvement de tissu adipeux humain provenant d'un enfant sain de moins de 10 ans,
b) récupération d'une fraction cellulaire dépourvue d'adipocytes, contenant tous les types cellulaires présents dans la préparation obtenue selon (a), à l'exception des adipocytes,
c) culture *in vitro* pendant au moins 12 heures, de la fraction cellulaire obtenue selon l'étape (b),
d) sélection des deux sous-populations cellulaires dites « CA » et « CS », la population « CA » présentant une vitesse d'adhésion inférieure à 12 heures, et la population « CS » présentant une vitesse d'adhésion supérieure à 12 heures,
e) enrichissement de la population « CA » jusqu'à l'obtention d'une population de cellules capables d'entrer dans un état de quiescence,
f) induction d'une prolifération des cellules souches de la population « CA ».

14. Procédé selon la revendication 12 ou 13, **caractérisé en ce que** le prélèvement de tissu adipeux est un prélèvement de tissu extramédullaire, provenant par exemple de la région ombilicale ou de la région pubienne ou de la région inguinale ou de la région périnéale ou de la région abdominale, ou de la région sous-cutanée.

15. Procédé selon la revendication 13, **caractérisé en ce que** la prolifération induite selon l'étape (f) est une prolifération intensive induite par addition du facteur de croissance fibroblastique basique (bFGF).

16. Procédé selon la revendication 13, **caractérisé en ce que** la digestion enzymatique selon l'étape (a) s'effectue par mise en contact du prélèvement de tissu adipeux avec une préparation de collagénase pendant une durée maximum de 10 minutes.

17. Procédé selon la revendication 13, **caractérisé en ce que** la fraction cellulaire dépourvue d'adipocytes est obtenue par la mise en oeuvre d'une étape d'élimination des adipocytes, par exemple par centrifugation.

18. Procédé selon la revendication 13, **caractérisé en ce que** la fraction cellulaire mise en culture selon l'étape (c) ne subit aucune étape de filtration avant d'être mise en culture.

19. Procédé selon la revendication 13, **caractérisé en ce que** l'étape de culture (c) s'effectue dans un milieu de culture additionné de sérum foetal sans ajout d'autres facteurs de croissance.

20. Procédé selon la revendication 13, **caractérisé en ce que**, lors de l'étape de culture (e), un transfert cellulaire est effectué lorsque les cellules arrivent à 80% de confluence, le transfert étant effectué à une densité d'ensemencement d'environ 1000 à 3500 cellules / cm².

21. Procédé selon la revendication 12 ou 13, **caractérisé en ce que** la population « CA » devient quiescente après environ 60 doublements de population.

22. Procédé selon la revendication 13, **caractérisé en ce qu'**il comprend en outre une étape d'obtention d'une population clonale à partir d'une cellule souche isolée à partir de la population « CA » obtenue après l'induction de la prolifération des cellules souches.

23. Population de cellules souches susceptible d'être obtenue par la mise en oeuvre du procédé selon l'une quelconque des revendications 12 à 22.

24. Population de cellules selon la revendication 23 **caractérisée en ce qu'**elle est clonale.

25. Population de cellules selon la revendication 23 ou 24, **caractérisée en ce qu'**elle est capable de proliférer en présence du facteur de croissance fibroblastique basique (bFGF).

26. Cellules souches selon l'une quelconque des revendications 1 à 11 ou population de cellules souches selon la revendication 23, 24 ou 25, pour une utilisation en thérapie.

27. Cellules souches ou population de cellules souches selon la revendication 26 **caractérisées en ce que** la thérapie comporte la transplantation de cellules chez un individu, suivie de la différenciation des cellules et la régénération de tissu *in vivo.*

28. Cellules souches ou population de cellules souches selon la revendication 27 **caractérisées en ce que** la transplantation est allogénique.

29. Cellule selon l'une quelconque des revendications 1 à 11, ou population de cellules selon la revendication 23 ou 25, pour une utilisation dans une méthode pour la régénération de tissu osseux, adipeux, musculaire ou endothélial *in vivo.*

30. Procédé pour la production de cellules différenciées du lignage mésodermique, **caractérisé en ce que** l'on cultive des cellules souches selon l'une quelconque des revendications 1 à 11, ou une population de cellules souches selon la revendication 23, 24 ou 25, à partir de la confluence, en présence d'un milieu de différenciation.

31. Procédé selon la revendication 30, **caractérisé en ce que** les cellules souches sont ensemencées à une densité d'environ 10 000 à 25 000 cellules / cm².

32. Procédé selon la revendication 30 ou 31, **caractérisé en ce que** le milieu de culture est un milieu permettant la différenciation en adipocytes.

33. Procédé selon la revendication 30 ou 31, **caractérisé en ce que** le milieu de culture est un milieu permettant la différenciation en ostéoblastes.

34. Procédé selon la revendication 30 ou 31, **caractérisé en ce que** le milieu de culture est un milieu permettant la différenciation en myocytes, ou un milieu angiogénique.

35. Procédé de criblage permettant d'identifier des agents susceptibles de moduler la différenciation de cellules en cellules du lignage mésodermique, **caractérisé par** :
a) la mise en culture de cellules souches selon l'une quelconque des revendications 1 à 11, ou d'une population de cellules souches selon la revendication 23, 24 ou 25, dans des conditions permettant leur différenciation en cellules du lignage mésodermique, en présence d'un agent candidat,
b) comparaison de la différenciation des cellules en présence de l'agent candidat avec la différenciation en l'absence de l'agent candidat.

36. Procédé selon la revendication 35 **caractérisé en ce que** les conditions de culture permettent la différenciation en adipocytes.

37. Procédé selon la revendication 35 **caractérisé en ce que** les conditions de culture permettent la différenciation en ostéoblastes.

38. Procédé selon la revendication 35 **caractérisé en ce que** les conditions de culture permettent la différenciation en myocytes.

39. Procédé selon la revendication 35 **caractérisé en ce que** l'agent susceptible de moduler la différenciation est une substance anti-différenciatrice.

40. Procédé de criblage permettant d'identifier des agents susceptibles de présenter une activité lipolytique, **caractérisé par** :
a) la mise en culture de cellules souches selon l'une quelconque des revendications 1 à 11, ou d'une population de cellules souches selon la revendication 23, 24 ou 25, dans des conditions permettant leur différenciation en adipocytes,
b) mise en contact des adipocytes ainsi obtenus, avec un agent candidat,
c) évaluation de l'activité lipolytique de l'agent candidat.

41. Procédé de criblage permettant d'identifier des agents susceptibles de présenter une activité anti-lipolytique, **caractérisé par** :
a) la mise en culture de cellules souches selon l'une quelconque des revendications 1 à 11, ou d'une population de cellules souches selon la revendication 23, 24 ou 25, dans des conditions permettant leur différenciation en adipocytes,
b) mise en contact des adipocytes ainsi obtenus, avec un agent candidat, en présence d'un agent lipolytique,
c) évaluation de l'activité anti-lipolytique de l'agent candidat.

42. Procédé de criblage permettant d'identifier des agents susceptibles de présenter une activité insulino-sensibilisante, **caractérisé par** :
a) la mise en culture de cellules souches selon l'une quelconque des revendications 1 à 11, ou d'une population de cellules souches selon la revendication 23, 24 ou 25, dans des conditions permettant leur différenciation en adipocytes,
b) mise en contact des adipocytes ainsi obtenus, avec un agent candidat,
c) évaluation de l'activité insulino-sensibilisante de l'agent candidat.

43. Utilisation des cellules souches selon l'une quelconque des revendications 1 à 11, ou d'une population de cellules souches selon la revendication 23, 24 ou 25, dans la cosmétique.

44. Composition cosmétique comprenant une pluralité de cellules selon l'une quelconque des revendications 1 à 11, ou d'une population de cellules souches selon la revendication 23, 24 ou 25, en association avec un excipient, véhicule, solvant, colorant, parfum, antibiotique ou autres additifs acceptables dans les produits cosmétiques.

45. Composition pharmaceutique comprenant une pluralité de cellules selon l'une quelconque des revendications 1 à 11, ou d'une population de cellules souches selon la revendication 23, 24 ou 25, en association avec un excipient acceptable du point de vue physiologique.

## Patentansprüche

1. Multipotente und adult isolierte humane Stammzelle, **dadurch gekennzeichnet, dass** sie:
i) eine endogene Telomeraseaktivität von wenigstens 20% der Telomeraseaktivität der transformierten humanen Linie HEK293T,
ii) einen HLA-Klasse-I-negativ Phänotyp, einschließlich in Gegenwart von 10% fötalem Kälberserum,
iii) einen normalen Karyotyp,
iv) eine Fähigkeit nach 50 bis 80 Populationsverdoppelungen ruhende zu werden,
v) eine während wenigstens 130 Populationsverdoppelungen konservierte Autoerneuerungskapazität und
vi) eine Seneszenzrate von weniger als 0,05% im Stadium von 60 Populationsverdoppelungen aufweist.

2. Stammzelle gemäß Anspruch 1, **dadurch gekennzeichnet, dass** sie eine während wenigstens 200 Populationsverdoppelungen konservierte Autoerneuerungskapazität besitzt.

3. Stammzelle gemäß Anspruch 1 oder 2, **dadurch gekennzeichnet, dass** sie aus humanem Fettgewebe isoliert werden kann.

4. Stammzelle gemäß einem jeden der vorhergehenden Ansprüche, **dadurch gekennzeichnet, dass** sie sich in eine Zelle endodermischen oder ektodermischen oder mesodermischen Ursprungs ausdifferenzieren kann.

5. Stammzelle gemäß Anspruch 4, **dadurch gekennzeichnet, dass** sie sich in Adipozyten, Osteoblasten, Myozyten, Chondrozyten oder in Endothelialzellen ausdifferenzieren kann.

6. Stammzelle gemäß einem jeden der vorhergehenden Ansprüche, **dadurch gekennzeichnet, dass** sie den Transkriptionsfaktor Oct-4 und/oder Rex-1 exprimiert.

7. Stammzelle gemäß einem jeden der vorhergehenden Ansprüche, **dadurch gekennzeichnet, dass** sie wenigstens ein Transgen exprimieren kann.

8. Stammzelle gemäß einem jeden der Ansprüche 1 bis 7, **dadurch gekennzeichnet, dass** sie den folgenden Phänotyp aufweist:
HLA-Klasse-I-negativ
HLA-Klasse-II-negativ
CD3 negativ
CD13 positiv

9. Zelle gemäß einem jeden der Ansprüche 1 bis 8, **dadurch gekennzeichnet, dass** sie in Gegenwart von 10% fötalem Kälberserum einen Phänotyp CD13-positiv aufweist.

10. Zelle gemäß Anspruch 8, **dadurch gekennzeichnet, dass** sie nach Erreichen des Stillstands in stabiler Form in vitro den nachfolgenden Phänotyp aufweist:
HLA-Klasse-I-negativ,
HLA-Klasse-II-negativ,
CD3 negativ,
CD13 positiv,
LIF-R-negativ,
Oct-4-positiv,
Rex-1 positiv, und
ABCG2-positiv.

11. Zelle gemäß Anspruch 10, **dadurch gekennzeichnet, dass** sie in vivo eine Fähigkeit besitzt, in den nicht-differenzierten Zustand zu migrieren und mehr als 10 Tage nach Transplantation in einem immunkompetenten Säuger keine Abstoßungsreaktion verursacht.

12. Verfahren zum Erhalten multipotenter humaner Stammzellen, umfassend die folgenden Schritte:
- Kultivieren von Zellen, welche von einer Probennahme aus humanem Fettgewebe stammen,
- Selektieren von zwei zellulären Unterpopulationen, genannt Population "CA" und Population "CS", wobei die Population "CA" eine Adhäsionsgeschwindigkeit von unter 12 Stunden und die Population "CS" eine Adhäsionsgeschwindigkeit von über 12 Stunden aufweist,
- Anreicherung der Population "CA" bis zum Erhalten einer Population von ruhenden Zellen,
- Induktion einer Proliferation von Stammzellen der Population "CA".

13. Verfahren gemäß Anspruch 12, umfassend die folgenden Schritte:
a) Enzymatisches Verdauen einer Probennahme von humanem Fettgewebe, das von einem gesunden Kind von weniger als 10 Jahren stammt,
b) Gewinnen einer von Adipozyten freien Zellfraktion, welche alle Zelltypen enthält, die gemäß (a) erhalten wurden, mit Ausnahme von Adipozyten,
c) in vitro Kultivieren der in Schritt (b) erhaltenen Zellfraktion während wenigstens 12 Stunden,
d) Selektieren von zwei zellulären Unterpopulationen, genannt "CA" und "CS", wobei die Population "CA" eine Adhäsionsgeschwindigkeit von unter 12 Stunden und die Population "CS" eine Adhäsionsgeschwindigkeit von über 12 Stunden aufweist,
e) Anreichern der Population "CA", bis zum Erhalten einer Zellpopulation, welche in den Ruhezustand eintreten kann,
f) Induktion einer Proliferation von Stammzellen der Population "CA".

14. Verfahren gemäß Anspruch 12 oder 13, **dadurch gekennzeichnet, dass** die Probennahme von Fettgewebe eine Probennahme von extramedulärem Gewebe ist, welches beispielsweise aus dem Nabelbereich oder dem Schambeinbereich oder dem Leistenbereich oder dem Dammbereich oder dem Abdomenbereich oder dem Subkutanbereich stammt.

15. Verfahren gemäß Anspruch 13, **dadurch gekennzeichnet, dass** die gemäß Schritt (f) induzierte Proliferation eine intensive Proliferation ist, welche durch den basischen Fibroplastenwachstumsfaktor (bFGF) induziert wird.

16. Verfahren gemäß Anspruch 13, **dadurch gekennzeichnet, dass** die enzymatische Verdauung gemäß Schritt (a) durch Inkontaktbringen der Probennahme von Fettgewebe mit einem Collagenase-Präparat während einer maximalen Dauer von 10 Minuten bewirkt wird.

17. Verfahren gemäß Anspruch 13, **dadurch gekennzeichnet, dass** die von Adipozyten freie Zellfraktion durch einen Schritt der Eliminierung von Adipozyten, beispielsweise durch Zentrifugieren, durchgeführt wird.

18. Verfahren gemäß Anspruch 13, **dadurch gekennzeichnet, dass** die in Schritt (c) kultivierte Zellfraktion keinerlei Filtration vor der Kultivierung unterworfen wird.

19. Verfahren gemäß Anspruch 13, **dadurch gekennzeichnet, dass** der Schritt der Kultivierung (c) in einem Kulturmedium, dem fötales Serum zugesetzt ist, ohne Zugabe weiterer Wachstumsfaktoren bewirkt wird.

20. Verfahren gemäß Anspruch 13, **dadurch gekennzeichnet, dass** während des Schrittes der Kultivierung (e) ein Zelltransfer bewirkt wird, wenn die Zellen 80% Konfluenz erreichen, wobei der Transfer bei einer Impfdichte von etwa 1000 bis 3500 Zellen/cm² bewirkt wird.

21. Verfahren gemäß Anspruch 12 oder 13, **dadurch gekennzeichnet, dass** die Population "CA" nach etwa 60 Populationsverdoppelungen ruhende wird.

22. Verfahren gemäß Anspruch 13, **dadurch gekennzeichnet, dass** es des Weiteren einen Schritt zum Erhalten einer klonalen Population aus einer aus der Population "CA" isolierten Stammzelle, welche nach Induktion der Proliferation von Stammzellen erhalten wurde, umfasst.

23. Population von Stammzellen, welche durch den Einsatz des Verfahrens gemäß einem jeden der Ansprüche 12 bis 22 erhalten werden kann.

24. Population von Zellen gemäß Anspruch 23, **dadurch gekennzeichnet, dass** sie klonal ist.

25. Population von Zellen gemäß Anspruch 23 oder 24, **dadurch gekennzeichnet, dass** sie in Gegenwart des basischen Fibroplastenwachstumsfaktors (bFGF) proliferieren kann.

26. Stammzellen gemäß einem jeden der Ansprüche 1 bis 11 oder Population von Stammzellen gemäß den Ansprüchen 23, 24 oder 25 zur Verwendung bei der Therapie.

27. Stammzellen oder Population von Stammzellen gemäß Anspruch 26, **dadurch gekennzeichnet, dass** die Therapie die Transplantation von Zellen in ein Individuum umfasst, gefolgt von der Ausdifferenzierung der Zellen und der Regeneration von Gewebe in vivo.

28. Stammzellen oder Population von Stammzellen gemäß Anspruch 27, **dadurch gekennzeichnet, dass** die Transplantation allogen ist.

29. Zelle gemäß einem jedem der Ansprüche 1 bis 11 oder Population von Zellen gemäß den Ansprüchen 23 oder 25 zur Verwendung bei einem Verfahren zur in vivo Regenerierung von Knochen-, Fett-, Muskel- oder Endothelialgewebe.

30. Verfahren zur Herstellung von differenzierten Zellen mesodermer Abstammung, **dadurch gekennzeichnet, dass** man Stammzellen gemäß einem jeden der Ansprüche 1 bis 11 oder eine Population von Stammzellen gemäß den Ansprüchen 23, 24 oder 25 aus der Konfluenz in Gegenwart eines Ausdifferenzierungsmilieus kultiviert.

31. Verfahren gemäß Anspruch 30, **dadurch gekennzeichnet, dass** die Stammzellen bei einer Dichte von etwa 10.000 bis 25.000 Zellen/cm² beimpft werden.

32. Verfahren gemäß Anspruch 30 oder 31, **dadurch gekennzeichnet, dass** das Kulturmilieu ein Milieu ist, welches die Ausdifferenzierung in Adipozyten erlaubt.

33. Verfahren gemäß Anspruch 30 oder 31, **dadurch gekennzeichnet, dass** das Kulturmilieu ein Milieu ist, das die Ausdifferenzierung in Osteoblasten erlaubt.

34. Verfahren gemäß Anspruch 30 oder 31, **dadurch gekennzeichnet, dass** das Kulturmilieu ein Milieu ist, welches die Ausdifferenzierung in Myozyten erlaubt, oder ein angiogenes Milieu.

35. Klassierungsverfahren, welches es gestattet, Stoffe zu identifizieren, welche die Ausdifferenzierung von Zellen in Zellen mesodermer Abstammung modulieren können, **gekennzeichnet durch**:
a) Kultivieren von Stammzellen gemäß einem jeden der Ansprüche 1 bis 11 oder einer Population von Stammzellen gemäß Anspruch 23, 24 oder 25, unter Bedingungen, welche ihre Differenzierung in Zellen mesodermer Abstammung in Gegenwart eines Kandidat-Stoffes erlauben,
b) Vergleich der Differenzierung von der Zellen in Gegenwart des Kandidat-Stoffes mit der Ausdifferenzierung ohne den Kandidat-Stoff.

36. Verfahren gemäß Anspruch 35, **dadurch gekennzeichnet, dass** die Kulturbedingungen die Ausdifferenzierung in Adipozyten erlauben.

37. Verfahren gemäß Anspruch 35, **dadurch gekennzeichnet, dass** die Kulturbedingungen die Ausdifferenzierung in Osteoplasten erlauben.

38. Verfahren gemäß Anspruch 35, **dadurch gekennzeichnet, dass** die Kulturbedingungen die Ausdifferenzierung in Myozyten erlauben.

39. Verfahren gemäß Anspruch 35, **dadurch gekennzeichnet, dass** der zum Modulieren der Ausdifferenzierung geeignete Stoff eine anti-ausdifferenzierende Substanz ist.

40. Klassierungsverfahren, welches die Identifizierung von Stoffen erlaubt, die eine lipolytische Wirkung aufweisen können, **gekennzeichnet durch**:
a) Kultivieren von Stammzellen gemäß einem jeden der Ansprüche 1 bis 11 oder einer Population von Stammzellen gemäß Anspruch 23, 24 oder 25, unter Bedingungen, welche ihre Differenzierung in Adipozyten erlauben,
b) Inkontaktbringen der so erhaltenen Adipozyten mit einem Kandidat-Stoff,
c) Evaluieren der lipolytischen Aktivität des Kandidat-Stoffes.

41. Klassierungsverfahren, welches die Identifizierung von Stoffen erlaubt, welche eine antilipolytische Aktivität aufweisen können, **gekennzeichnet durch**:
a) Kultivieren von Stammzellen gemäß einem jeden der Ansprüche 1 bis 11 oder einer Population von Stammzellen gemäß Anspruch 23, 24 oder 25, unter Bedingungen, welche ihre Differenzierung in Adipozyten erlauben,
b) Inkontaktbringen der so erhaltenen Adipozyten mit einem Kandidat-Stoff in Gegenwart eines lipolytischen Stoffes,
c) Evaluieren der anti-lipolytischen Aktivität des Kandidat-Stoffes.

42. Klassierungsverfahren, welches die Identifizierung eines Stoffes erlaubt, welcher eine insulinsensibilisierende Aktivität aufweisen kann, **gekennzeichnet durch**:
a) Kultivieren von Stammzellen gemäß einem jeden der Ansprüche 1 bis 11 oder einer Population von Stammzellen gemäß Anspruch 23, 24 oder 25, unter Bedingungen, welche ihre Differenzierung in Adipozyten erlaubt,
b) Inkontaktbringen der so erhaltenen Adipozyten mit einem Kandidat-Stoff,
c) Evaluieren der insulinsensibilisierenden Aktivität des Kandidat-Stoffes.

43. Verwendung von Stammzellen gemäß einem jeden der Ansprüche 1 bis 11 oder einer Population von Stammzellen gemäß Anspruch 23, 24 oder 25 in der Kosmetik.

44. Kosmetische Zusammensetzung, umfassend eine Vielzahl von Zellen gemäß einem jeden der Ansprüche 1 bis 11 oder eine Population von Stammzellen gemäß Anspruch 23, 24 oder 25 in Verbindung mit einem Exzipienten, Träger, Lösemittel, Farbstoff, Parfüm, Antibiotikum oder weiteren für kosmetische Produkte annehmbare Zusatzstoffe.

45. Pharmazeutische Zusammensetzung, umfassend eine Vielzahl von Zellen gemäß einem jeden der Ansprüche 1 bis 11 oder eine Population von Stammzellen gemäß Anspruch 23, 24 oder 25 in Verbindung mit einem aus physiologischer Sicht annehmbaren Exzipienten.

## Claims

1. An adult multipotent human stem cell, **characterized in that** it has:
i) a telomerase activity of at least 20% of the telomerase activity of the transformed human line HEK293T,
ii) a negative HLA Class I phenotype, including in the presence of 10% fetal calf serum,
iii) a normal caryotype,
iv) a capacity to become quiescent after 50 to 80 population doublings,
v) a capacity for self-renewal preserved for at least 130 population doublings, and
vi) a senescence rate lower than 0.05% at 60 population doublings.

2. Stem cell according to claim 1, **characterized in that** it has a self-renewal capacity preserved for at least 200 population doublings.

3. Stem cell according to claim 1 or 2, **characterized in that** it can be isolated from human adipose tissue.

4. Stem cell according to any one of the preceding claims, **characterized in that** it can differentiate into cells of endodermal, ectodermal or mesodermal origin.

5. Stem cell according to claim 4, **characterized in that** it is capable of differentiating into an adipocyte, osteoblast, myocyte, chondrocyte or endothelial cell.

6. Stem cell according to any one of the preceding claims, **characterized in that** it expresses the transcription factor Oct-4 and/or Rex-1.

7. Stem cell according to any one of the preceding claims, **characterized in that** it can express at least one transgene.

8. Stem cell according to any one of claims 1 to 7, **characterized in that** it has the following phenotype:
negative HLA class I
negative HLA class II
negative CD3
positive CD13

9. Stem cell according to any one of claims 1 to 8, **characterized in that** it has a positive CD13 phenotype in the presence of 10% of foetal calf serum.

10. Cell according to claim 8, **characterized in that** after reaching quiescence, it has the following stable phenotype:
negative HLA class I,
negative HLA class II,
negative CD3,
positive CD13,
negative LIF-R,
positive Oct-4,
positive Rex-1, and
positive ABCG2.

11. Cell according to claim 10, **characterized in that** in vivo it has a capacity to migrate in the undifferentiated state, and does not give rise to a rejection reaction more than 10 days following transplantation in an immunocompetent mammal.

12. A method for obtaining multipotent human stem cells comprising the following steps:
- culturing cells from a human adipose tissue sample,
- selecting two cell sub-populations termed a "CA" population and "CS" population, the "CA" population having an adhesion rate of less than 12 hours, and the "CS" population having an adhesion rate of more than 12 hours,
- enriching the "CA" population until a quiescent cell population is obtained,
- inducing proliferation of stem cells of the "CA" population.

13. A method according to claim 12, comprising the following steps:
a) enzymatic digestion of a sample of human adipose tissue deriving from a healthy child under 10 years of age;
b) recovering a cell fraction that is free of adipocytes, containing all of the cell types present in the preparation obtained in (a) with the exception of adipocytes;
c) carrying out in vitro culture of the cell fraction obtained in step (b) for at least 12 hours;
d) selecting of two cell sub-populations termed "CA" and "CS", the "CA" population having an adhesion rate of less than 12 hours, and the "CS" population having an adhesion rate of more than 12 hours;
e) enriching the "CA" population until a population of cells is obtained that is capable of entering a quiescent state,
f) inducing proliferation of stem cells of the "CA" population.

14. Method according to claim 12 or 13, **characterized in that** the adipose tissue sample is a sample of extramedullary tissue derived, for example, from the umbilical region or from the pubic region or from the inguinal region or from the perineal region or from the abdominal region or from the subcutaneous region.

15. Method according to claim 13, **characterized in that** the proliferation induced in step (f) is intensive proliferation induced by adding basic fibroblast growth factor (bFGF).

16. Method according to claim 13, **characterized in that** enzymatic digestion in step (a) is carried out by bringing the adipose tissue sample into contact with a collagenase preparation for a maximum period of 10 minutes.

17. Method according to claim 13, **characterized in that** the cell fraction that is free of adipocytes is obtained by carrying out an adipocyte elimination step, for example by centrifugation.

18. Method according to claim 13, **characterized in that** the cell fraction that is cultured in step (c) does not undergo any filtration steps before culturing.

19. Method according to claim 13, **characterized in that** the culture step (c) is carried out in a culture medium supplemented with foetal calf serum without the addition of other growth factors.

20. Method according to claim 13, **characterized in that** during culture step (e), cell transfer is carried out when the cells are at 80% confluence, transfer being carried out at a seeding density of about 1000 to 3500 cells/cm².

21. Method according to claim 12 or 13, **characterized in that** the "CA" population becomes quiescent after about 60 population doublings.

22. Method according to claim 13, **characterized in that** it further comprises a step of obtaining a clonal population from a stem cell isolated from the "CA" population obtained after the induction of stem cell proliferation.

23. Population of stem cells obtainable by carrying out the method according to any one of claims 12 to 22.

24. Cell population according to claim 23, **characterized in that** it is clonal.

25. Cell population according to claim 23 or 24, **characterized in that** it is capable of proliferating in the presence of basic fibroblast growth factor (bFGF).

26. Stem cells according to any one of claims 1 to 11 or population of stem cells according to claim 23, 24 or 25, for use in therapy.

27. Stem cells or population of stem cells according to claim 26, **characterized in that** the therapy comprises transplantation of cells into an individual followed by cell differentiation and tissue regeneration in vivo.

28. Stem cells or population of stem cells according to claim 27, **characterized in that** transplantation is allogenic.

29. Cell according to any one of claims 1 to 11, or cell population according to claim 23 or 25, for use in a method for regenerating bone, adipose, muscle or endothelial tissue *in vivo.*

30. A method for producing differentiated mesodermal cells, **characterized in that** stem cells according to any one of claims 1 to 11 or a population of stem cells according to claim 23, 24 or 25, are cultivated from confluence in the presence of a differentiation medium.

31. Method according to claim 30, **characterized in that** the stem cells are seeded at a density of about 10 000 to 25 000 cells/cm².

32. Method according to claim 30 or 31, **characterized in that** the culture medium is a medium allowing differentiation into adipocytes.

33. Method according to claim 30 or 31, **characterized in that** the culture medium is a medium allowing differentiation into osteoblasts.

34. Method according to claim 30 or 31, **characterized in that** the culture medium is a medium allowing differentiation into myocytes, or an angiogenic medium.

35. A screening method to identify agents that can modulate the differentiation of cells into mesodermal cells, **characterized by** :
a) culturing stem cells according to any one of claims 1 to 11, or a population of stem cells according to claim 23, 24 or 25, under conditions that allow their differentiation into mesodermal cells, in the presence of a candidate agent;
b) comparing the differentiation of cells in the presence of a candidate agent with differentiation in the absence of the candidate agent.

36. Method according to claim 35, **characterized in that** the culture conditions allow differentiation into adipocytes.

37. Method according to claim 35, **characterized in that** the culture conditions allow differentiation into osteoblasts.

38. Method according to claim 35, **characterized in that** the culture conditions allow differentiation into myocytes.

39. Method according to claim 35, **characterized in that** the agent that can modulate differentiation is an anti-differentiation substance.

40. A screening method for identifying agents that may have a lipolytic activity, **characterized by** :
a) culturing stem cells according to any one of claims 1 to 11, or a population of stem cells according to claim 23, 24 or 25, under conditions allowing their differentiation into adipocytes,
b) bringing the adipocytes obtained into contact with a candidate agent,
c) evaluating the lipolytic activity of the candidate agent.

41. A screening method for identifying agents that may have an anti-lipolytic activity, **characterized by** :
a) culturing stem cells according to any one of claims 1 to 11, or a population of stem cells according to claim 23, 24 or 25, under conditions allowing their differentiation into adipocytes,
b) bringing the adipocytes obtained into contact with a candidate agent,
c) evaluating the anti-lipolytic activity of the candidate agent.

42. A screening method for identifying agents that may have an insuline-sensitizing activity, **characterized by** :
a) culturing stem cells according to any one of claims 1 to 11, or a population of stem cells according to claim 23, 24 or 25, under conditions allowing their differentiation into adipocytes,
b) bringing the adipocytes obtained into contact with a candidate agent,
c) evaluating the insuline-sensitizing activity of the candidate agent.

43. Use of stem cells according to any one of claims 1 to 11, or a population of stem cells according to claim 23, 24 or 25, in cosmetics.

44. A cosmetic composition comprising a plurality of cells according to any one of claims 1 to 11, or a population of stem cells according to claim 23, 24 or 25, in association with an excipient, vehicle, solvent, colorant, fragrance, antibiotic or other additives acceptable in cosmetic products.

45. A pharmaceutical composition comprising a plurality of cells according to any one of claims 1 to 11, or a population of stem cells according to claim 23, 24 or 25, in association with a physiologically acceptable excipient.
